(19) Europäisches Patentamt
European Patent Office
Office européen des brevets

(11) EP 3 561 175 A1

(12) **EUROPEAN PATENT APPLICATION**
published in accordance with Art. 153(4) EPC

(43) Date of publication:
**30.10.2019 Bulletin 2019/44**

(21) Application number: **17885911.2**

(22) Date of filing: **25.12.2017**

(51) Int Cl.:
***D06M 13/256*** *(2006.01)*
***C11D 1/14*** *(2006.01)*
***D06L 1/16*** *(2006.01)*
***D06M 13/463*** *(2006.01)*
***D06M 15/643*** *(2006.01)*
***C07C 309/20*** *(2006.01)*
***D06M 101/04*** *(2006.01)*

(86) International application number:
**PCT/JP2017/046375**

(87) International publication number:
**WO 2018/123942 (05.07.2018 Gazette 2018/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **26.12.2016 JP 2016250991**

(71) Applicant: **Kao Corporation**
**Chuo-Ku**
**Tokyo 103-8210 (JP)**

(72) Inventors:
- **MORIKAWA, Satoshi**
**Wakayama-shi**
**Wakayama 640-8580 (JP)**
- **KUSUNOKI, Ayako**
**Wakayama-shi**
**Wakayama 640-8580 (JP)**

(74) Representative: **Hoffmann Eitle**
**Patent- und Rechtsanwälte PartmbB**
**Arabellastraße 30**
**81925 München (DE)**

(54) **METHOD FOR PROCESSING FIBER PRODUCT**

(57) The present invention is a method for washing a textile product, including the following step 1 and step 2:
step 1: a step of treating the textile product by contacting the textile product with treating liquid 1 obtained by mixing water and (A) an internal olefin sulfonate having 17 or more and 24 or less carbons [hereinafter, referred to as component (A)]; and
step 2: a step of treating the textile product treated in step 1 by contacting the textile product with treating liquid 2 obtained by mixing water and a finishing agent composition for textile products.

EP 3 561 175 A1

## Description

Field of the Invention

The present invention relates to a method for treating a textile product, a method for producing a textile product, a method for retaining water absorbency of a textile product, a water absorbency retaining agent for textile products, and a function modifier for a finishing base for textile products.

Background of the Invention

Anionic surfactants, due to excellent detergent property and foaming power thereof, have conventionally been widely used as domestic detergent ingredients. As one of anionic surfactants other than alkylbenzene sulfonates, an olefin sulfonate, particularly, an internal olefin sulfonate obtained by using an internal olefin having a double bond inside the olefin chain, not at its end, as raw material, has been reported. A method for washing a textile product, including washing the textile product by use of a detergent composition containing an anionic surfactant followed by softening the textile product with a softener composition for textile products, is also conducted in ordinary households.

JP-A 2011-47065 discloses a method for washing a textile product including washing a textile product with a detergent containing a specific non-ionic surfactant and an anionic surfactant in a specific ratio followed by conducting a stored-water rinsing step in which the textile product is treated with rinse water containing a textile product treatment agent, the method providing an excellent water-saving property without compromising the detergency and softness.

JP-A 2014-76988 discloses an internal olefin sulfonate composition containing (A) an internal olefin sulfonate having 16 carbons and/or (B) an internal olefin sulfonate having 18 carbons, the composition having a content mass ratio (A/B) between component (A) and component (B) of 0/100 to 70/30, and having excellent foamability, foam quality, rapid foamability, and foam disappearing property, in particular, foam quality, during hair washing.

EP-A 377261 discloses a detergent composition containing an internal olefin sulfonate containing a β-hydroxy form of 25% or more, the composition having excellent detergency. EP-A 377261 mentions that the internal olefin sulfonate preferably has 12 to 20 carbons. Specifically, a method for treating stains attached to polyester/cotton fabric products is disclosed.

JP-A 3-126793 describes a detergent composition containing an internal olefin sulfonate derived from an internal olefin having 12 to 18 carbons and a nonionic surfactant having an HLB value of 10.5 or less in a specific ratio. The example describes evaluation on washing in water of which ionic strength is adjusted with sodium chloride and sodium carbonate, by use of an internal olefin sulfonate having 18 carbons as a comparative example.

JP-A 6-123071 describes a softening finishing agent containing an amine compound that has a hydrocarbon containing one unsaturated group and has a ratio of total cis forms/total trans forms in the stereoisomeric structure = 25/75 to 90/10, or a neutral salt or quaternary salt thereof as an essential component. JP-A 6-123071 mentions that the agent exhibits a satisfactory softening effect on various fibers and additionally improves the water absorbing capacity of fibers markedly.

Summary of the Invention

The present invention provides a method for treating a textile product, the method being capable of retaining water absorbency possessed by the textile product.

The present invention also provides a method for producing a textile product, the method enabling production of a textile product having excellent water absorbency.

The present invention also provides a method for retaining water absorbency possessed by a textile product.

The present invention also provides a water absorbency retaining agent for textile products, the agent retaining water absorbency possessed by the textile product.

The present invention also provides a method for modifying a function of a finishing base for textile products.

The present invention also provides a function modifier for a finishing base for textile products.

The present inventors have found that treating a textile product with an internal olefin sulfonate having a hydrocarbon group having a specific number of carbons in advance before treating the textile product with a finishing agent composition for textile products enables to retain the water absorbency inherently possessed by the textile products before treatment. The present inventors have also found an application of an internal olefin sulfonate having a hydrocarbon group with a specific number of carbons as an agent for retaining inherent water absorbency of a textile product. The present inventors have also found an application of an internal olefin sulfonate having a hydrocarbon group with a specific number of carbons as a function modifier for a finishing base for fibers.

The present invention relates to a method for treating a textile product, including the following step 1 and step 2:

step 1: a step of treating the textile product by contacting the textile product with treating liquid 1 obtained by mixing water and (A) an internal olefin sulfonate having 17 or more and 24 or less carbons; and
step 2: a step of treating the textile product treated in step 1 by contacting the textile product with treating liquid 2 obtained by mixing water and a finishing agent composition for textile products.

The present invention also relates to a method for producing a textile product, including the following step 1 and step 2:

step 1: a step of treating the textile product by contacting the textile product with treating liquid 1 obtained by mixing water and (A) an internal olefin sulfonate having 17 or more and 24 or less carbons; and
step 2: a step of treating the textile product treated in step 1 by contacting the textile product with treating liquid 2 obtained by mixing water and a finishing agent composition for textile products.

The present invention also relates to a method for retaining water absorbency of a textile product, wherein, in treating the textile product by steps including the following step 1 and step 2, (A) an internal olefin sulfonate having 17 or more and 24 or less carbons is employed as an anionic surfactant of step 1:

step 1: a step of treating the textile product by contacting the textile product with treating liquid 1 obtained by mixing water and the anionic surfactant; and
step 2: a step of treating the textile product treated in step 1 by contacting the textile product with treating liquid 2 obtained by mixing water and a finishing agent composition for textile products.

The present invention also relates to a water absorbency retaining agent for textile products, containing (A) an internal olefin sulfonate having 17 or more and 24 or less carbons.
The present invention also relates to a method for modifying a function of a finishing base for textile products, wherein, in treating a textile product by steps including the following step 1 and step 2, (A) an internal olefin sulfonate having 17 or more and 24 or less carbons is employed as an anionic surfactant of step 1:

step 1: a step of treating the textile product by contacting the textile product with treating liquid 1 obtained by mixing water and the anionic surfactant; and
step 2: a step of treating the textile product treated in step 1 by contacting the textile product with treating liquid 2 obtained by mixing water and a finishing agent composition for textile products containing a finishing base for textile products.

The present invention also relates to a function modifier for a finishing base for textile products, containing (A) an internal olefin sulfonate having 17 or more and 24 or less carbons.
(A) Internal olefin sulfonate having 17 or more and 24 or less carbons will be described as component (A) hereinbelow.
According to the present invention, it is possible to retain water absorbency inherently possessed by a textile product before treatment, even when the product is treated with any type of finishing agent composition for textile products. According to the present invention, it is also possible to modify a finishing function of textile products, possessed by a finishing base for textile products.

Embodiments of the Invention

<Component (A)>

Component (A) of the present invention is an internal olefin sulfonate having 17 or more and 24 or less carbons. Component (A) has an effect of retaining water absorbency inherent in fibers. In the present invention, retaining the water absorbency inherent in fibers means that water absorbency 2/water absorbency 1 is 0.72 or more and 1.0 or less, for example. Water absorbency 1 herein is the water absorbency of a textile product after the textile product is treated, for example, washed only with water each in step 1, without use of component (A), and in step 2, without use of a finishing agent composition for textile products. Water absorbency 2 is the water absorbency of a textile product after the textile product is treated, for example, washed by the method of the present invention. Specifically, water absorbency 1 and water absorbency 2 can be determined by the method in examples.
Component (A) also has an effect of modifying a function of a finishing base for textile products to thereby inhibit decrease in water absorbency of a textile product treated with the finishing base for textile products. In the present invention, modifying a function of a finishing base for textile products includes, for example, modifying the finishing base

for textile products such that changes in water absorbency inherent in textile products are inhibited by the finishing base for textile products while the function of finishing textile products possessed by the finishing base for textile products is retained. In the present invention, modifying a function of finishing base for textile products means that water absorbency 2/water absorbency 1 is 0.72 or more and 1.0 or less, for example. Water absorbency 1 herein is the water absorbency of a textile product after the textile product is treated, for example, washed only with water each in step 1, without use of component (A), and in step 2, without use of a finishing agent composition for textile products containing a finishing base for textile products. Water absorbency 2 is water absorbency of a textile product after the textile product is treated, for example, washed by the method of the present invention.

The action mechanism of component (A) of the present invention is not necessarily evident, but it is assumed that conducting step 1 of treating a textile product using component (A) of the present invention, before step 2 of treating the textile product with a finishing base for textile products such as component (B) described below, allows component (A) slightly remaining in the textile product to modify, in step 2, a state in which the finishing base for textile products adsorbs to the textile product into a state in which changes in the water absorbency of the textile product can be inhibited, while an ability of imparting a texture to the textile product, possessed by the finishing base for textile products, is retained.

The number of carbons of the internal olefin sulfonate in component (A) represents the number of carbons of the internal olefin to which the sulfonate is covalently attached. The number of carbons of an internal olefin sulfonate having 17 or more and 24 or less carbons is 17 or more, preferably 18 or more, and 24 or less, preferably 22 or less, more preferably 20 or less, further preferably 19 or less, from the viewpoint that water absorbency inherent in a textile product can be retained.

The internal olefin sulfonate of the present invention is a sulfonate obtained by sulfonating, neutralizing, and hydrolyzing an internal olefin (olefin having a double bond inside an olefin chain) having 17 or more and 24 or less carbons as raw material.

Such an internal olefin may also contain a trace amount of so-called an alpha-olefin (hereinafter, also referred to as an $\alpha$-olefin), in which the position of the double bond is located at position 1 of the carbon chain. When the internal olefin is sulfonated, a $\beta$-sultone is quantitatively generated. A portion of the $\beta$-sultone changes into a $\gamma$-sultone and then an olefin sulfonate, and these are further converted into a hydroxyalkane sulfonate and an olefin sulfonate in a neutralization/hydrolysis step (e.g., J. Am. Oil Chem. Soc. 69, 39 (1992)). The hydroxy group of the hydroxyalkane sulfonate obtained herein is located inside the alkane chain, and the double bond of the olefin sulfonate is located inside the olefin chain. The product obtained is mainly a mixture of these, a portion of which may contain a trace amount of a hydroxyalkane sulfonate having a hydroxy group at the end of the carbon chain or an olefin sulfonate having a double bond at the end of the carbon chain.

These products and mixtures thereof herein each are collectively referred to as an internal olefin sulfonate (component (A)). Additionally, the hydroxyalkane sulfonate is referred to as the hydroxy form of the internal olefin sulfonate (hereinafter, also referred to as HAS), and the olefin sulfonate is referred to as the olefin form of the internal olefin sulfonate (hereinafter, also referred to as IOS).

The mass ratio between HAS and IOS in component (A) may be measured with a high performance liquid chromatography-mass spectrometer (hereinafter, abbreviated as an HPLC-MS). Specifically, the mass ratio can be determined from the HPLC-MS peak areas of component (A).

Examples of the internal olefin sulfonate of component (A) include alkali metal salts, alkaline earth metal (1/2 atom) salts, ammonium salts, and organic ammonium salts. Examples of the alkali metal salt include Na salts and K salts. Examples of the organic ammonium salt include alkanol ammonium salts having 2 or more and 6 or less carbons.

The content of internal olefin sulfonates having the sulfonate group at position 5 or higher in the component (A) is, from the viewpoint of being capable of further benefiting from effects of the present invention, preferably 10% by mass or more, more preferably 10.5% by mass or more, further preferably 11.0% by mass or more, furthermore preferably 12.0% by mass or more, furthermore preferably 12.5% by mass or more, furthermore preferably 13.0% by mass or more, furthermore preferably 13.5% by mass or more, and preferably 60% by mass or less, more preferably 55% by mass or less, further preferably 50% by mass or less, furthermore preferably 45% by mass or less, furthermore preferably 40% by mass or less, furthermore preferably 35% by mass or less, furthermore preferably 30% by mass or less.

The content of the olefin sulfonate including the sulfonate group located at position 1 in component (A) is limited from the viewpoint of being capable of retaining water absorbency inherent in a textile product before treatment even if the textile product is treated with a finishing agent composition for textile products. The content thereof in component (A) is preferably 10% by mass or less, more preferably 7% by mass or less, further preferably 5% by mass or less, furthermore preferably 3% by mass or less, and from the viewpoint of reduction in the production cost and enhancement in the productivity, preferably 0.01% by mass or more.

The position of the sulfonate group of these compounds is the position in the olefin chain or in the alkane chain.

In respect that water absorbency inherent in a textile product can be retained by controlling the state of adsorption of a finishing agent composition for textile products to the textile product even when any type of finishing agent composition for textile products is used, it is preferred that component (A) of the present invention contain an internal olefin sulfonate

having 17 or more and 24 or less carbons with the sulfonate group located at position 2 or higher and position 4 or lower (IO-1S) and an internal olefin sulfonate having 17 or more and 24 or less carbons with the sulfonate group located at position 5 or higher (IO-2S) and that the mass ratio of (IO-1S) to (IO-2S), (IO-1S)/(IO-2S), be 0.6 or more and 6 or less.

That is, in respect that water absorbency inherent in a textile product can be retained by controlling the state of adsorption of a finishing agent composition for textile products to the textile product even when any type of finishing agent composition for textile products is used, component (A) of the present invention is preferably an internal olefin sulfonate having 17 or more and 24 or less carbons, wherein the mass ratio of the internal olefin sulfonate having 17 or more and 24 or less carbons with the sulfonate group located at position 2 or higher and position 4 or lower (IO-1S) to the internal olefin sulfonate having 17 or more and 24 or less carbons with the sulfonate group located at position 5 or higher (IO-2S) in the internal olefin sulfonate, (IO-1S)/(IO-2S), is 0.6 or more and 6 or less.

(IO-1S)/(IO-2S), as the mass ratio of the content of (IO-1S) to the content of (IO-2S) in component (A) is, in respect that water absorbency inherent in a textile product can be retained by controlling the state of adsorption of a finishing agent composition for textile products to the textile product even when any type of finishing agent composition for textile products is used, preferably 0.6 or more, more preferably 0.65 or more, further preferably 0.70 or more, furthermore preferably 0.75 or more, furthermore preferably 0.8 or more, furthermore preferably 1.0 or more, furthermore preferably 1.2 or more, furthermore preferably 1.4 or more, furthermore preferably 1.6 or more, furthermore preferably 2.0 or more, furthermore preferably 2.5 or more, furthermore preferably 3.0 or more, furthermore preferably 3.5 or more, furthermore preferably 4.0 or more, and from the viewpoint of being unlikely to impair the texture imparting effect of the finishing agent composition for textile products, preferably 6 or less, more preferably 5.9 or less, further preferably 5.8 or less, furthermore preferably 5.7 or less, furthermore preferably 5.6 or less, furthermore preferably 5.5 or less, furthermore preferably 5.4 or less, furthermore preferably 5.3 or less, furthermore preferably 5.2 or less, furthermore preferably 5.1 or less, furthermore preferably 5.0 or less, furthermore preferably 4.9 or less, furthermore preferably 4.8 or less, furthermore preferably 4.7 or less.

The content of each compound having the sulfonate group at a different position in component (A) may be determined with a high performance liquid chromatography-mass spectrometer (hereinafter, abbreviated as an HPLC-MS). The content of each compound having the sulfonate group at a different position herein is to be determined as the mass ratio based on HPLC-MS peak areas of the compounds having the sulfonate group at each position in all the HAS's of component (A).

The HAS herein is the hydroxy form of internal olefin sulfonate, that is, hydroxyalkanesulfonate among compounds produced by the sulfonation of the internal olefin sulfonic acid.

Specifically, the content of each compound having the sulfonate group at a different position in component (A) may be determined with a high performance liquid chromatography-mass spectrometer (hereinafter, abbreviated as an HPLC-MS). The content of each compound having the sulfonate group at a different position herein is to be determined as a mass ratio based on HPLC-MS peak areas of the compounds having the sulfonate group at each position in all the HAS forms of component (A). The HAS herein is a hydroxyalkane sulfonate, that is the hydroxy form of the internal olefin sulfonate among compounds to be produced by sulfonating an internal olefin sulfonate. In the present invention, the content of the internal olefin sulfonate having 17 or more and 24 or less carbons with the sulfonate group located at each position takes the numerical value based on the HPLC-MS peak area of the sulfonate having 17 or more and 24 or less carbons with sulfonate group located at each position, among the HAS forms having 17 or more and 24 or less carbons, as its representative value.

In the present invention, the content of the internal olefin sulfonate having 17 or more and 24 or less carbons with the sulfonate group located at position 2 or higher and position 4 or lower (IO-1S) takes the numerical value based on the HPLC-MS peak area of the sulfonate having 17 or more and 24 or less carbons with the sulfonate group located at position 2 or higher and position 4 or lower, among the HAS forms having 17 or more and 24 or less carbons, as its representative value.

Additionally, in the present invention, the content of the internal olefin sulfonate having 17 or more and 24 or less carbons with the sulfonate group located at position 5 or higher takes the numerical value based on the HPLC-MS peak area of the sulfonate having 17 or more and 24 or less carbons with the sulfonate group located at position 5 or higher, among the HAS forms having 17 or more and 24 or less carbons, as its representative value.

The internal olefin sulfonate as component (A) may be constituted by including an internal olefin sulfonate having 17 or more and 24 or less carbons with the sulfonate group located at position 2 or higher and position 4 or lower (IO-1S) and an internal olefin sulfonate having 17 or more and 24 or less carbons with the sulfonate group located at position 5 or higher (IO-2S). The maximum value of the position to which the sulfonate group is bonded in the internal olefin sulfonate (IO-2S) depends on the number of carbons.

The mass ratio (IO-1S)/(IO-2S) in component (A) is based on the component (A) finally obtained. For example, even if the internal olefin sulfonate is one obtained by mixing an internal olefin sulfonates having the mass ratio (IO-1S)/(IO-2S) out of the above range, the sulfonate is intended to correspond to the preferable internal olefin sulfonate of component (A) as long as the mass ratio (IO-1S)/(IO-2S) in the composition of the internal olefin sulfonate is in the above

range.

The internal olefin sulfonate can be a mixture of the hydroxy form and the olefin form. The mass ratio of the content of the olefin form of the internal olefin sulfonate to the content of the hydroxy form of the internal olefin sulfonate (olefin form/hydroxy form) in component (A) can be 0/100 or more, further 5/95 or more, and 50/50 or less, further 40/60 or less, further 30/70 or less, further 25/75 or less.

The mass ratio of the content of the hydroxy form of the internal olefin sulfonate to the content of the olefin form of the internal olefin sulfonate in component (A) can be determined by the method described in examples after component (A) is separated into the hydroxy form and the olefin form by HPLC.

Component (A) can be produced by sulfonation, neutralization and hydrolysis of an internal olefin having 17 or more and 24 or less carbons that is a raw material.

The sulfonation can be carried out by reacting 1.0 to 1.2 mol of sulfur trioxide gas with 1 mol of the internal olefin. The reaction may be carried out at 20 to 40°C.

The neutralization is carried out by reacting an alkali aqueous solution such as sodium hydroxide, ammonia, and 2-aminoethanol in an amount of 1.0 to 1.5 molar times the theoretical value of the sulfonate group.

The hydrolysis may be carried out at 90 to 200°C for 30 minutes to 3 hours in the presence of water.

These reactions can be continuously carried out. After the completion of the reactions, purification can be carried out by extraction, washing or the like.

When producing the internal olefin sulfonate of component (A), the treatment such as sulfonation, neutralization, and hydrolysis may be performed using a raw material internal olefin having a carbon number distribution within the range of 17 or more and 24 or less. Alternatively, the treatment such as sulfonation, neutralization, and hydrolysis may be performed using a raw material internal olefin having a single carbon atom. Alternatively, a plurality of types of internal olefin sulfonates having different numbers of carbons that have been produced in advance as required may be mixed.

In the present invention, the internal olefin is an olefin having a double bond inside the olefin chain, as described above. The internal olefin as the raw material of component (A) has 17 or more and 24 or less carbons. One type of internal olefin may be used for component (A), or two or more types may be used in combination.

The total content of an olefin having a double bond at position 1, so-called an alpha olefin, in the raw material internal olefin is, from the viewpoint of being capable of retaining water absorbency inherent in a textile product before treatment even if the textile product is treated with a finishing agent composition for textile products, preferably 10% by mass or less, more preferably 7% by mass or less, further preferably 5% by mass or less, furthermore preferably 3% by mass or less, and, from the viewpoint of reduction in the production cost and enhancement in the productivity, preferably 0.01% by mass or more, in component (A).

The content of an olefin having a double bond located at position 5 or higher in the raw material internal olefin is, from the viewpoint of being capable of retaining water absorbency inherent in a textile product before treatment even if the textile product is treated with a finishing agent composition for textile products, preferably 10% by mass or more, and preferably 60% by mass, more preferably 55% by mass or less, further preferably 50% by mass or less, furthermore preferably 45% by mass or less, furthermore preferably 40% by mass or less, furthermore preferably 35% by mass or less, furthermore preferably 30% by mass. From the viewpoint of the improvement in the detergency against stains attached to fibers, the content is preferably 60% by mass, more preferably 55% by mass or less, further preferably 50% by mass or less, furthermore preferably 45% by mass or less. The maximum value of the position of the double bond in the raw material internal olefin depends on the number of carbons.

Distribution of the double bond in the raw material internal olefin can be determined, for example, by a gas chromatograph mass spectrometer (hereinafter abbreviated as GC-MS). Specifically, each component different in the carbon chain length and the double bond position is precisely separated from each other by a gas chromatograph analyzer (hereinafter abbreviated as GC), each component can be subjected to a mass spectrometer (hereinafter abbreviated as MS) to identify the double bond position, and the proportion of each component can be determined from its GC peak area. The method for determining the position of the double bond in the raw material internal olefin is shown below.

<Method for measuring double bond position in raw material internal olefin>

The position of the double bond in the internal olefin is measured by gas chromatography (hereinafter abbreviated as GC). Specifically, the internal olefin is allowed to react with dimethyl disulfide to form its dithiolated derivative, and each component is then subjected to separation by GC. As a result, the position of the double bond of the internal olefin is determined from each of the resulting peak areas.

The devices and the analysis conditions used for the measurement are as follows:

GC apparatus: "HP6890" (manufactured by Hewlett Packard Company);
column: "Ultra-Alloy-1 HT Capillary column" (30 m × 250 μm × 0.15 μm, manufactured by Frontier Laboratories, Inc.);

detector (hydrogen flame ionization detector (FID));
injection temperature: 300°C;
detector temperature: 350°C; and
He flow rate: 4.6 mL/min.

In the present invention, as the mass of component (A), the value of the counter ion calculated based on the form of a sodium ion is used.

<Finishing agent composition for textile products>

Subsequently, a finishing agent composition for textile products to be used in step 2 will be described.
The finishing agent composition for textile products to be used in the present invention preferably contains a finishing base for textile products as component (B). More specific examples of component (B) include one or more components selected from (B1) cationic surfactants having a hydrocarbon group having 16 or more and 24 or less [hereinafter, may be referred to as component (B1)], (B2) silicone compounds [hereinafter, may be referred to as component (B2)], (B3) fatty alcohols [hereinafter may be referred to as component (B3)], (B4) fatty acid esters [may be referred to as component (B4)], (B5) fats and oils [hereinafter may be referred to as component (B5)], and (B6) fatty acids or salts thereof [hereinafter, may be referred to as component (B6)].
Examples of the cationic surfactant as component (B1) include cationic surfactants as tertiary amine salts and cationic surfactants as quaternary ammonium salts. Examples of the cationic surfactant as component (B1) include compounds represented by the following general formula (I):

[Formula 1]

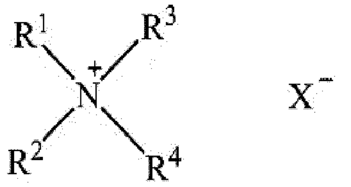

(1)

wherein $R^1$ is a hydrocarbon group having a total of 16 or more and 24 or less carbons, which may be interrupted by an ester group and/or amide group; $R^2$ and $R^3$ are each independently a hydrocarbon group having a total of 16 or more and 24 or less carbons, which may be interrupted by an ester group and/or amide group, a hydroxyalkyl group having 1 or more and 3 or less carbons, or an alkyl group having 1 or more and 3 or less carbons; $R^4$ is a hydrogen atom or an alkyl group having 1 or more and 3 or less carbons; and $X^-$ is an anionic group.
In general formula (1), $R^1$ is a hydrocarbon group having a total of 16 or more and 24 or less, from the viewpoint of enhancement in softness, preferably 16 or more and 22 or less, further preferably 16 or more and 20 or less carbons, which may be interrupted by an ester group and/or amide group. The hydrocarbon group is a saturated hydrocarbon group or unsaturated hydrocarbon group. $R^2$ and $R^3$ are each independently a hydrocarbon group having a total of 16 or more and 24 or less, from the viewpoint of enhancement in softness, preferably 16 or more and 22 or less, further preferably 16 or more and 20 or less carbons, which may be interrupted by a hydroxyalkyl group having 1 or more and 3 or less carbons, preferably a hydroxyethyl group, an alkyl group having 1 or more and 3 or less carbons, preferably a methyl group, an ethyl group, or an ester group and/or an amide group. The hydrocarbon group is a saturated hydrocarbon group or an unsaturated hydrocarbon group. $R^4$ is a hydrogen atom or an alkyl group having 1 or more and 3 or less carbons. $X^-$ is a halogen ion, a sulfate ion, a fatty acid having 1 or more and 12 or less carbons, an alkyl sulfate ion having 1 or more and 3 or less carbons, or the like.
In respect of being capable of further benefiting from an effect of retaining water absorbency of textile products of the present invention, the hydrocarbon group having a total of 16 or more and 24 or less carbons, which may be interrupted by an ester group and/or amide group in $R^1$, $R^2$, and $R^3$ is preferably a hydrocarbon group containing a saturated hydrocarbon group.
The content of the saturated hydrocarbon group in the total amount of the saturated hydrocarbon group and unsaturated hydrocarbon group, in a total of 16 or more and 24 or less carbons, which may be interrupted by an ester group and/or amide group in the cationic surfactant represented by general formula (1) is, from the viewpoint of being capable of further benefiting from effects of the present invention, preferably more than 0% by mass, more preferably 1% by mass or more, further preferably 3% by mass or more, furthermore preferably 5% by mass or more, furthermore preferably 10% by mass or more, furthermore preferably 20% by mass or more, furthermore preferably 30% by mass

or more, furthermore preferably 40% by mass or more, furthermore preferably 50% by mass or more, furthermore preferably 60% by mass or more, furthermore preferably 70% by mass or more, furthermore preferably 80% by mass or more, furthermore preferably 85% by mass or more, furthermore preferably 90% by mass or more, furthermore preferably 95% by mass or more, furthermore preferably 97% by mass or more, furthermore preferably 99% by mass or more, and preferably 100% by mass or less. The content of the saturated hydrocarbon group in the total amount of the saturated hydrocarbon group and unsaturated hydrocarbon group contained in the cationic surfactant represented by general formula (1) may be 100% by mass.

It is generally known that a cationic surfactant containing a larger amount of the saturated hydrocarbon group in the hydrocarbon group having a total of 16 or more and 24 or less carbons, which may be interrupted by an ester group and/or amide group, is more likely to lower water absorbency inherent in fibers. In the present invention, even with use of component (B1), which seems to have a relatively large degree of lowering water absorbency inherent in a textile product, treating the textile product with component (A) before treating the textile product with component (B1) can bring the water absorbency closer to that inherent in a textile product.

The compound represented by general formula (1) is produced by selecting a composition of the hydrocarbon group of a fatty acid or a fatty acid lower alkyl (1 or more and 3 or less carbons in the alkyl group) ester and subjecting a specific number of moles of the raw material fatty acid or the fatty acid lower alkyl (1 or more and 3 or less carbons in the alkyl group) ester having the composition and a corresponding alkanolamine to dehydration esterification or transesterification, and can be produced by quaternization by use of an alkylating agent or neutralization by use of an acid agent.

Examples of the above alkanolamine include, but are not limited to, dialkyl monoalkanolamines, preferably dimethyl monoethanolamine and dimethyl monopropanolamine, monoalkyl dialkanolamines, preferably methyl diethanolamine and methyl dipropanolamine, and trialkanolamines, preferably triethanolamine and tripropanolamine. More preferable alkanolamines are trialkanolamines.

In order to control the content of the unsaturated hydrocarbon group having two or more double bonds in the raw material fatty acid or fatty acid lower alkyl ester, for example, crystallization as described in JP-A 4-306296, a method of distilling methyl ester under reduced pressure as described in JP-A 6-41578, selective hydrogenation as described in JP-A 8-99036, and the like can be conducted.

In esterification or transesterification, the molar ratio between the fatty acid or fatty acid lower alkyl ester and the hydroxy group of the alkanolamine is, as fatty acid or fatty acid lower alkyl ester:hydroxy group of the alkanolamine, preferably 0.3:1.0 or more, further preferably 0.5:1.0 or more, and preferably 1.2:1.0 or less, further preferably 1.0:1.0 or less.

Examples of the alkylating agent include dialkyl sulfates (1 or more and 3 or less carbons in the alkyl groups) and halogenated alkyls (1 or more and 3 or less carbons in the alkyl group). Examples of the acid agent include inorganic acids and organic acids. Examples of the inorganic acids include hydrochloric acid and sulfuric acid. Examples of the organic acids include salts of carboxylic acids having 2 or more and 6 or less carbons, such as lactic acid, glycolic acid, and citric acid. Other examples of the organic acids include alkyl sulfates having 1 or more and 3 or less carbons.

The quaternization by use of an alkylating agent may be conducted in the presence of a solvent (e.g., ethanol), but synthesis is more preferably conducted in the absence of a solvent from the viewpoint of inhibiting generation of impurities. The neutralization by use of an acid agent may be conducted either in the presence of water or in the presence of a solvent (e.g., ethanol).

As the mass of the cationic surfactant as component (B1) used herein, the mass of the counter ion calculated based on the form of a methyl sulfate is to be used.

Among components (B), as a silicone compound as component (B2), one or more silicone compounds selected from dimethyl silicone, amino-modified silicone, and polyether-modified silicone are preferred. From the viewpoint of being capable of further benefiting from effects of the present invention, one or more silicone compounds selected from dimethyl silicone and polyether-modified silicone are preferred.

As the dimethyl silicone, a dimethylpolysiloxane having a viscosity of 1000 to 600000 $mm^2/s$ at 25°C is preferred. The viscosity is a kinematic viscosity measured with an Ubbelohde viscometer (manufactured by SIBATA SCIENTIFIC TECHNOLOGY LTD.) in a temperature range of 25°C$\pm$0.2°C.

As the amino-modified silicone, preferred is an amino-modified silicone having a kinematic viscosity at 25°C (hereinafter, the viscosity of the amino-modified silicone means this viscosity, unless otherwise indicated) of 100 to 20000 $mm^2/s$ and an amino equivalent of 400 to 8000 g/mol. The kinematic viscosity at 25°C can be determined with an Ostwald viscometer. The kinematic viscosity is preferably 100 $mm^2/s$ or more, more preferably 200 $mm^2/s$ or more, further preferably 500 $mm^2/s$ or more, from the viewpoint of an enhanced effect of imparting a texture to textile products, and preferably 20000 $mm^2/s$ or less, more preferably 10000 $mm^2/s$ or less, further more preferably 5000 $mm^2/s$ or less, in respect of being more likely to benefit from effects of the present invention.

The amino equivalent of the amino-modified silicone is preferably 400 g/mol or more, more preferably 600 g/mol or more, further preferably 800 g/mol or more, from the viewpoint of being capable of further benefiting from effects of the present invention and preferably 8000 g/mol or less, more preferably 5000 g/mol or less, further preferably 4000

g/mol or less, from the viewpoint of imparting a texture to textile products. The amino equivalent is a molecular weight per nitrogen atom, is determined by amino equivalent = molecular weight/number of nitrogen atoms, and can be determined by the % by weight of nitrogen that can be determined by elementary analysis.

As the polyether-modified silicone, a polyether-modified silicone having an HLB of more than 0 and 7 or less is preferred. As the polyether-modified silicone having an HLB of more than 0 and 7 or less, the compound of general formula (4) and the compound of general formula (5) described in paragraph number 0024 to 0035 of JP-A 2007-131989 may be used.

Examples of the compound of general formula (4) described in JP-A 2007-131989 mentioned above include FZ-2203, FZ-2206, FZ-2207, and FZ-2222 manufactured by ex-Nippon Unicar Company Limited (manufactured by the present Dow Corning Toray Silicone Co. Ltd.). Examples of the compound of general formula (5) described in JP-A 2007-131989 mentioned above include SH3772M, SH3775M, and FZ-2233 manufactured by Dow Corning Toray Silicone Co. Ltd. and KF6012, KF6016, and KF6017 manufactured by Shin-Etsu Chemical Co., Ltd.

In the present invention, component (B) may include one or more components (B1) and one or more components (B2). In other words, the finishing agent composition for textile products may contain one or more cationic surfactants (B1) and one or more silicone compounds (B2) as component (B). In this case, the mass ratio of component (B2)/component (B1) is preferably 0.01 or more, more preferably 0.05 or more, and preferably 0.1 or less, more preferably 1 or less.

Examples of the fatty alcohol as component (B3) include, from the viewpoint of allowing textile products to have a smoother finish, fatty alcohols having preferably 10 or more, more preferably 12 or more, further preferably 14 or more carbons, and preferably 22 or less, more preferably 18 or less carbons. Specific examples of the fatty alcohol include saturated or unsaturated fatty alcohols. Specifically, examples thereof can include decyl alcohol, myristyl alcohol, palmityl alcohol, stearyl alcohol, oleyl alcohol, linoleyl alcohol, isocetyl alcohol, isostearyl alcohol, behenyl alcohol, hexadecyl alcohol, phenylethyl alcohol, cetanol, olelyl alcohol, 2-octyl dodecanol, batyl alcohol, and 2-hexyl decanol.

Examples of the fatty acid ester as component (B4) include, from the viewpoint of allowing textile products to have a smoother finish, fatty acid esters having a molecular weight of 300 or more and 3000 or less. Specifically, preferred are ester compounds of a fatty acid having 10 or more and 18 or less carbons and a monovalent or higher and hexavalent or lower alcohol having 1 or more and 6 or less carbons. Specific and preferable examples of the alcohol can include monovalent alcohols selected from ethanol, isopropanol, butanol, and hexanol, divalent alcohols such as ethylene glycol, propylene glycol, diethylene glycol, dipropylene glycol, and 1,6-hexanediol, and trivalent or higher alcohols such as glycerin (in the case of glycerin, the ester is a monoester or diester), pentaerythritol, glucose, sorbitol, and sorbitan. Component (B5) is excluded from component (B4) .

Examples of the fat and oil as component (B5) include vegetable and animal fats and oils and hydrides thereof, from the viewpoint of allowing textile products to have a smoother finish. Specifically, component (B5) is preferably one or more selected from olive oil, avocado oil, evening primrose oil, jojoba oil, camellia oil, macadamia nut oil, peppermint oil, sunflower oil, rapeseed oil, sesame oil, wheat germ oil, castor oil, safflower oil, cottonseed oil, soybean oil, jojoba oil, coconut oil, palm oil, palm kernel oil, beef tallow, lard, fish oil, horse oil, egg yolk oil, carnauba wax, and lanolin.

Examples of fatty acid or salt thereof as component (B6) include, from the viewpoint of allowing textile products to have a smoother finish, saturated or unsaturated fatty acids having preferably 12 or more carbons, more preferably 14 or more carbons and preferably 18 or less carbons, and salts thereof. Specific examples of the fatty acid can include lauric acid, myristic acid, palmitic acid, palmitoleic acid, stearic acid, oleic acid, linoleic acid, or salts thereof. Examples of the salt can include inorganic metal salts, further, alkali metal salts such as sodium salts.

Among components (B1) to (B6), from the viewpoint of enabling the user to further realize the effect of retaining water absorbency inherent in a textile product before treatment, one or more compounds selected from component (B1), component (B2), component (B3), component (B4), and component (B6) are preferable, and one or more compounds selected from component (B1), component (B2), and component (B4) are more preferable.

<Textile product>

From the viewpoint that, even if a textile product is treated with a finishing agent composition for textile products, the user is more likely to realize the effect of retaining water absorbency inherent in the textile product before treatment in the case of component (A) of the present invention than in the case of an anionic surfactant conventionally known as a detergent ingredient, fibers are preferably textile products containing hydrophilic fibers.

Examples of the hydrophilic fiber include seed hair fibers (such as cotton, cotton, and kapok), bast fibers (such as hemp, linen, ramie, cannabis, and jute), vein fibers (such as abaca and sisal), palm fibers, rush, straw, animal hair fibers (such as wool, mohair, cashmere, camel hair, alpaca, vicuna, and Angora), silk fibers (house silkworm silk, wild silkworm silk), down, and cellulosic fibers (such as rayon, polynosic, cupro, and acetate).

Fibers constituting textile products covered in the present invention may contain hydrophobic fibers in addition to the hydrophilic fibers described above, as long as effects of the present invention are not impaired. Examples of the hydrophobic fiber include protein-based fibers (such as milk protein casein fiber and promix), polyamide-based fibers

(such as nylon), polyester-based fibers (such as polyester), polyacrylonitrile-based fibers (such as acryl), polyvinyl alcohol-based fibers (such as vinylon), polyvinyl chloride-based fibers (such as polyvinyl chloride), polyvinylidene chloride-based fibers (such as vinylidene), polyolefin-based fibers (such as polyethylene and polypropylene), polyurethane-based fibers (such as polyurethane), polyvinyl chloride/polyvinyl alcohol copolymer-based fibers (such as polychlal), polyalkylene paraoxybenzoate-based fibers (such as benzoate), polyfluoroethylene-based fibers (such as polytetrafluoroethylene), glass fibers, carbon fibers, alumina fibers, silicon carbide fibers, rock fibers, slag fibers, and metal fibers (gold threads, silver threads, and steel fibers).

In the present invention, textile products mean fabric such as woven fabric, knitted fabric, or nonwoven fabric produced by using the hydrophilic fibers or the hydrophilic fibers and optional hydrophobic fibers, and products obtained by using the same such as undershirts, T-shirts, business shirts, blouses, pants, hats, handkerchieves, towels, knits, socks, underwear, and tights.

From the viewpoint that, even if a textile product is treated with a finishing agent composition for textile products, the user is more likely to realize the effect of retaining water absorbency inherent in the textile product before treatment in the case of component (A) of the present invention than in the case of an anionic surfactant conventionally known as a detergent ingredient, the textile product preferably contains a hydrophilic fiber, and more preferably contains a cotton fiber. The content of the hydrophilic fiber in the textile product is, from the viewpoint of further enabling the water absorbency to be improved, preferably 5% by mass or more, more preferably 10% by mass or more, further preferably 15% by mass or more, furthermore preferably 20% by mass or more, furthermore preferably 100% by mass. The content of cotton fiber is, from the viewpoint of further enabling the water absorbency to be improved, preferably 5% by mass or more, more preferably 10% by mass or more, further preferably 15% by mass or more, furthermore preferably 20% by mass or more, furthermore preferably 100% by mass.

<Method of the present invention>

A method for treating a textile product, for example, a washing method of the present invention is a method for treating a textile product, for example, a washing method, including the following step 1 and step 2. An example of the method for treating a textile product of the present invention is a method for washing a textile product, including the following step 1 and step 2. Another example of the treating method of the present invention is a step of finishing a textile product in a step of producing the textile product.

step 1: a step of treating the textile product by contacting the textile product with treating liquid 1 obtained by mixing water and (A) an internal olefin sulfonate having 17 or more and 24 or less carbons; and
step 2: a step of treating the textile product treated in step 1 by contacting the textile product with treating liquid 2 obtained by mixing water and a finishing agent composition for textile products.

The method for producing a textile product of the present invention is a method for producing a textile product, including the following step 1 and step 2:

step 1: a step of treating the textile product by contacting the textile product with treating liquid 1 obtained by mixing water and (A) an internal olefin sulfonate having 17 or more and 24 or less carbons; and
step 2: a step of treating the textile product treated in step 1 by contacting the textile product with treating liquid 2 obtained by mixing water and a finishing agent composition for textile products.

A method for retaining water absorbency of a textile product of the present invention is a method for retaining water absorbency of a textile product, wherein, in treating the textile product by steps including the following step 1 and step 2, (A) an internal olefin sulfonate having 17 or more and 24 or less carbons is employed as an anionic surfactant of step 1:

step 1: a step of treating the textile product by contacting the textile product with treating liquid 1 obtained by mixing water and the anionic surfactant; and
step 2: a step of treating the textile product treated in step 1 by contacting the textile product with treating liquid 2 obtained by mixing water and a finishing agent composition for textile products.

According to the method for retaining water absorbency of a textile product of the present invention, it is possible to retain water absorbency inherently possessed by a textile product. The water absorbency herein can be evaluated by using, for example, the water absorbing rate of the textile product, the feeling felt when water can be wiped off in the case where the skin onto which water attaches is wiped with the textile product, the area of water spread at a certain time after water is dropped onto surface of the textile product, and the like to be described below as indices, or by using

the water absorbing rate of the textile product as an index.

The method for modifying a function of a finishing base for textile products of the present invention is a method for modifying a function of a finishing base for textile products, wherein, in treating the textile product by steps including the following step 1 and step 2, (A) an internal olefin sulfonate having 17 or more and 24 or less carbons is employed as an anionic surfactant of step 1:

step 1: a step of treating the textile product by contacting the textile product with treating liquid 1 obtained by mixing water and the anionic surfactant; and
step 2: a step of treating the textile product treated in step 1 by contacting the textile product with treating liquid 2 obtained by mixing water and a finishing agent composition for textile products containing a finishing base for textile products.

According to the method for modifying a function of a finishing base for textile products of the present invention, it is possible to modify the function of the finishing base for textile products by inhibiting influence on water absorbency inherent in a textile product by the finishing base for textile products, especially decrease in the water absorbency while the texture imparting effect inherently possessed by the finishing base for textile products is retained. The water absorbency herein can be evaluated by using, for example, the water absorbing rate of the textile product, the feeling felt when water can be wiped off in the case where the skin onto which water attaches is wiped with the textile product, and the water absorbing rate of the textile product to be described below as indices.

Hereinafter, step 1, step 2, optional steps, and the like will be described. Unless otherwise indicated, the following description can be applied to the four methods of the present invention.

<Step 1>

Step 1 is a step of contacting a textile product with water and an anionic surfactant to treat the textile product, wherein the anionic surfactant is the following component (A):
component (A): an internal olefin sulfonate having 17 or more and 24 or less carbons.

Component (A) and the textile product used in step 1 are the same as component (A) and the textile product mentioned above, respectively.

In step 1, water and the anionic surfactant containing at least component (A) are mixed to obtain treating liquid 1. The water used in step 1 may be referred to as water 1, hereinafter.

Water 1 is preferably water containing a hardness component such as calcium and magnesium, in respect of being capable of further benefiting from effects of retaining the water absorbency of a textile product, even if the textile product is treated with a finishing agent composition for textile products. When water containing a hardness component is used, the hardness of the water is, for example, 0.5°dH or more, more preferably 1°dH or more, further preferably 2°dH or more, furthermore preferably 3°dH or more, and preferably 20°dH or less, more preferably 10°dH or less, further preferably 8°dH or less, furthermore preferably 6°dH or less.

The German hardness (°dH) herein refers to the concentrations of calcium and magnesium in water expressed as the concentration calculated based on the form of $CaCO_3$: 1 mg/L (ppm) = about 0.056° dH (1°dH = 17.8 ppm) .

The concentrations of calcium and magnesium for this German hardness are determined by a chelate titration method using disodium ethylenediaminetetraacetate salt. A specific method for measuring the German hardness of water herein is described below.

<Method for measuring German hardness of water>

[Reagent]

- 0.01 mol/l EDTA·2Na solution: a 0.01 mol/l aqueous solution of disodium ethylenediaminetetraacetate (a titration solution, 0.01 M EDTA-Na2, manufactured by SIGMA-ALDRICH)
- Universal BT indicator (product name: Universal BT, manufactured by Dojindo Laboratories)
- Ammonia buffer solution for hardness measurement (a solution prepared by dissolving 67.5 g of ammonium chloride in 570 ml of 28 w/v% ammonia water and adding ion-exchanged water until the total volume is 1000 ml)

[Measurement of hardness]

(1) 20 ml of water as a sample is collected in a conical beaker using a whole pipette;
(2) 2 ml of an ammonia buffer solution for hardness measurement is added thereto;
(3) 0.5 ml of Universal BT indicator is added thereto, and it is made sure that the solution after addition is reddish violet;
(4) while the conical beaker is shaken well, a 0.01 mol/l EDTA-2Na solution is added dropwise thereto from a burette, and the point at which the sample water turns blue is taken as the end point of the titration; and
(5) the total hardness is determined by the following calculation formula:

$$\text{Hardness } (^{\circ}\text{dH}) = T \times 0.01 \times F \times 56.0774 \times 100/A$$

T: titer of a 0.01 mol/l EDTA-2Na solution (mL)
A: sample volume (20 mL, a volume of sample water), and
F: factor of a 0.01 mol/l EDTA-2Na solution.

In treating liquid 1 to be used in step 1, the content of component (A) in treating liquid 1 or the content of component (A) in the total mass of water 1 and component (A) is preferably 10 mg/kg or more, more preferably 30 mg/kg or more, further preferably 70 mg/kg or more, furthermore preferably 100 mg/kg or more, from the viewpoint of being capable of further washing off stains attached to a textile product or the viewpoint of being capable of retaining the water absorbency of the textile product obtained in step 1 even if step 2 described below is conducted, and preferably 5000 mg/kg or less, more preferably 3000 mg/kg or less, further preferably 2000 mg/kg or less, furthermore preferably 1000 mg/kg or less, from the viewpoint of being capable of retaining the water absorbency of the textile product obtained in step 1 even if step 2 described below is conducted.

The content of component (A) contained in treating liquid 1 is based on the value calculated by assuming that the counter ion is a sodium ion.

Water 1 and component (A) constitute a mixture in step 1. This mixture is treating liquid 1. The temperature of treating liquid 1 is, from the viewpoint of enabling the detergency against stains attached to the textile product to be further improved, preferably 0°C or more, more preferably 3°C or more, further preferably 5°C or more, and preferably 40°C or less, more preferably 35°C or less.

The pH of treating liquid 1 at 25°C is, from the viewpoint of improving the detergency against stains attached to fibers and from the viewpoint of being capable of retaining the water absorbency of the textile product obtained in step 1 even if step 2 described below is conducted, preferably 4 or more, more preferably 5 or more, and preferably 10.5 or less, more preferably 10 or less, further preferably 9 or less, furthermore preferably 8.5 or less. A specific method for measuring a pH in the present invention is described below.

<pH measurement method>

A pH measuring composite electrode (glass fitting sleeve-type, manufactured by HORIBA, Ltd.) is connected to a pH meter (pH/ion meter F-23, manufactured by HORIBA, Ltd.) and the power is turned on. A saturated potassium chloride aqueous solution (3.33 mol/L) is used as an internal liquid for pH electrodes. Next, a pH 4.01 standard solution (a phthalate standard solution), a pH 6.86 standard solution (a neutral phosphate standard solution), and a pH 9.18 standard solution (a borate standard solution) are each filled into a 100 mL beaker, and immersed in a thermostat bath at 25°C for 30 minutes. The pH measuring electrode is immersed for 3 minutes in each of the standard solutions adjusted to a constant temperature, and subjected to a calibration operation in the order of pH 6.86 → pH 9.18 → pH 4.01. Each of samples to be measured is adjusted to 25°C, the electrode of the pH meter is immersed in the sample, and the pH after 1 minute is measured.

In step 1, the value of the bath ratio expressed as the ratio of the volume of water 1 or treating liquid 1 (liter) to the mass of the textile product (kg), that is, volume of water 1 or treating liquid 1 (liter)/mass of textile product (kg) may be referred to as bath ratio 1. When bath ratio 1 decreases in use of a household washing machine, the amount of the anionic surfactant to be carried over to step 2 may increase. Even under a treatment condition including small bath ratio 1 in the method for treating a textile product in step 1, it is possible to inhibit decrease in the effect of a finishing agent composition for textile products to impart a texture to the textile product in step 2 described below and thus to retain the water absorbency inherently possessed by the textile product obtained in step 1. From the viewpoint of being capable of retaining the detergency against stains attached to the textile product, or of being capable of retaining, in step 2 described below, the water absorbency of the textile product obtained in step 1 while inhibiting the decrease in the effect of the finishing agent composition for textile products to impart a texture to the textile product even if the textile product is treated with a finishing agent composition for textile products in step 2 described below, bath ratio 1 is preferably 2 or more, more preferably 3 or more, further preferably 4 or more, furthermore preferably 5 or more, and preferably 45

or less, more preferably 40 or less, further preferably 30 or less, furthermore preferably 20 or less.

According to the present invention, from the viewpoint of easily removing stains attached to the textile product, or of being capable of retaining, in step 2 described below, the water absorbency inherent in the textile product obtained in step 1 even if the textile product is treated with a finishing agent composition for textile products, the treatment time in step 1 is preferably 1 minute or more, more preferably 2 minutes or more, further preferably 3 minutes or more, preferably 1 hour or less, more preferably 30 minutes or less, further preferably 20 minutes or less, furthermore preferably 15 minutes or less.

A rotary washing machine can be used for treatment of the textile product in step 1. Specific examples of the rotary washing machine include drum washing machines, pulsator washing machines, and agitator washing machines. As these rotary washing machines, those commercially available for household use may be each used. In respect of enabling further reduction in the amount of water used for one round of washing, drum washing machines, which have rapidly spread in recent years, can reduce the amount of water especially during treatment. The treatment on a textile product of the present invention is preferably treatment on a textile product by use of a drum washing machine in respect of being capable of further benefiting from effects of the present invention.

In step 1, it is also possible to treat the textile product by contacting treating liquid 1, which contains water and an anionic surfactant containing at least component (A), with the textile product followed by leaving treating liquid 1 to stand. Leaving treating liquid 1 to stand means a state in which the textile product and treating liquid 1 are contacted with each other and then left to stand. A stirring operation such as shaking and manual agitation may be conducted before or after the textile product and treating liquid 1 are left to stand, or furthermore, in the course of the textile product and treating liquid 1 being left to stand.

<Optional component>

Treating liquid 1 for use in step 1 of the present invention can contain components other than component (A) and water.

For treating liquid 1 for use in step 1, a surfactant other than component (A) and component (B) as component (C) can be used as long as effects of the present invention are not impaired. Examples of component (C) include one or more surfactants selected from anionic surfactants other than component (A) and non-ionic surfactants. Examples of component (C) include one or more surfactants selected from the following component (c1), component (c2), component (c3), component (c4), and component (c5):

component (c1): an alkyl or alkenyl sulfate
component (c2): a polyoxyalkylene alkyl ether sulfate or polyoxyalkylene alkenyl ether sulfate
component (c3): an anionic surfactant having a sulfonate group (except for component (A))
component (c4): a fatty acid or salt thereof
component (c5): a nonionic surfactant having a polyoxyalkylene group

More specific examples of component (c1) include one or more anionic surfactants selected from alkyl sulfates having an alkyl group having 10 or more and 18 or less carbons and alkenyl sulfates having an alkenyl group having 10 or more and 18 or less carbons. From the viewpoint of improving the detergency, component (c1) is preferably one or more anionic surfactants selected from alkyl sulfates having an alkyl group having 12 or more and 14 or less carbons, more preferably one or more anionic surfactants selected from sodium alkyl sulfates having an alkyl group having 12 or more and 14 or less carbons.

Specific examples of component (c2) include one or more anionic surfactants selected from polyoxyalkylene alkyl sulfate having an alkyl group having 10 or more and 18 or less carbons and an average number of added moles of alkylene oxide of 1 or more and 3 or less and polyoxyalkylene alkenyl ether sulfates having an alkenyl group having 10 or more and 18 or less carbons and an average number of added moles of alkylene oxide of 1 or more and 3 or less. From the viewpoint of improving the detergency, component (c2) is preferably a polyoxyethylene alkyl sulfate having an average number of added moles of ethylene oxide of 1 or more and 2.2 or less, more preferably a polyoxyethylene alkyl sulfate having an alkyl group having 12 or more and 14 or less carbons and an average number of added ethylene oxide of 1 or more and 2.2 or less, furthermore preferably sodium salts thereof.

An anionic surfactant having a sulfonate group as component (c3) refers to an anionic surfactant having a sulfonate as a hydrophilic group (except for component (A)).

Specific examples of component (c3) include one or more anionic surfactants selected from alkylbenzene sulfonates having an alkyl group having 10 or more and 18 or less carbons, alkenylbenzene sulfonates having an alkenyl group having 10 or more and 18 or less carbons, alkane sulfonates having an alkyl group having 10 or more and 18 or less carbons, $\alpha$-olefin sulfonates having an $\alpha$-olefin moiety having 10 or more and 18 or less carbons, $\alpha$-sulfofatty acid salts having a fatty acid moiety having 10 or more and 18 or less carbons, and $\alpha$-sulfofatty acid lower alkyl ester salts

having a fatty acid moiety having 10 or more and 18 or less carbons and an ester moiety having 1 or more and 5 or less carbons. From the viewpoint of improving the detergency, component (c3) is preferably an alkylbenzene sulfonate having an alkyl group having 11 or more and 14 or less carbons, more preferably a sodium alkylbenzene sulfonate having an alkyl group having 11 or more and 14 or less carbons.

Examples of the fatty acid or a salt thereof as component (c4) include fatty acids or salts thereof having 10 or more and 20 or less carbons. Component (c4) may have 12 or more, 14 or more, and 20 or less, 18 or less carbons.

Component (c5) is preferably a nonionic surfactant represented by the following general formula (c5):

$$R^{1c}(CO)_m\text{-}O\text{-}(AO)_n\text{-}R^{2c} \qquad (c5)$$

wherein $R^{1c}$ is an aliphatic hydrocarbon group having 9 or more and 16 or less carbons, $R^{2c}$ is a hydrogen atom or methyl group, CO is a carbonyl group, m is a number of 0 or 1, the AO group is one or more groups selected from an ethyleneoxy group and a propyleneoxy group, and n is an average number of added moles, which is a number of 3 or more and 100 or less.

In general formula (c5), $R^{1c}$ is an aliphatic hydrocarbon group having 9 or more and 16 or less carbons. In respect of easily removing stains attached to fibers, $R^1$ has 9 or more, preferably 10 or more, more preferably 11 or more, and 16 or less, preferably 15 or less, more preferably 14 or less carbons. The aliphatic hydrocarbon group as $R^{1c}$ is preferably a group selected from an alkyl group and alkenyl group.

In general formula (c5), the AO group is one or more groups selected from an ethyleneoxy group and a propyleneoxy group. When an ethyleneoxy group and a propyleneoxy group are included, the ethyleneoxy group and the propyleneoxy group may be block bonded or randomly bonded.

In general formula (c5), n is an average number of added moles, which is a number of 3 or more and 100 or less. In respect of easily removing stains attached to fibers, n is 3 or more, preferably 6 or more, more preferably 6.5 or more, further preferably 7 or more, furthermore preferably 8 or more, and 100 or less, preferably 60 or less, more preferably 50 or less, further preferably 40 or less, furthermore preferably 35 or less, furthermore preferably 30 or less.

Examples of the salt of an anionic surfactant as components (c1) to (c4) include alkali metal salts and alkanolamine salts having 2 or more and 6 or less carbons.

As for treating liquid 1 for use in step 1, from the viewpoint of being capable of further benefiting from effects of the present invention, the proportion of component (A) in the entire anionic surfactants contained in treating liquid 1 is preferably 50% by mass or more, more preferably 60% by mass or more, further preferably 70% by mass or more, and preferably 100% by mass or less, and may be 100% by mass.

As for treating liquid 1 for use in step 1, from the viewpoint of being capable of further benefiting from effects of the present invention, the proportion of component (A) in the entire surfactants contained in treating liquid 1 is preferably 55% by mass or more, more preferably 65% by mass or more, further preferably 75% by mass or more, and 100% by mass or less, and may be 100% by mass.

For treating liquid 1 for use in step 1, a metal ion chelating agent can be used as component (D) as long as effects of the present invention are not impaired. In the method for treating a textile product, for example, the washing method of the present invention, the textile product is preferably subjected to a treatment in which water containing a hardness component is employed. Thus, component (D) may not be formulated in the composition.

Component (D) is preferably a metal ion chelating agent that can chelate divalent or higher metal ions.

Examples of component (D) of the present invention include one or more metal ion chelating agents selected from (D1) a metal ion chelating agent as an inorganic compound and (D2) a metal ion chelating agent as an organic compound.

[(D1) Metal ion chelating agent as inorganic compound]

Examples of (D1) the metal ion chelating agent as an inorganic compound include one or more metal ion chelating agents selected from (D1-1) alkali metal silicates, (D1-2) aluminosilicates, and (D1-3) tripolyphosphates. Hereinafter, these components will be described.

(D1-1) The alkali metal silicate is an alkali metal salt of silicic acid ($SiO_2$), and a compound in which $SiO_2/M_2O$ (M represents an alkali metal) of the alkali metal silicate is 0.5 or more and 2.6 or less is generally employed. More specifically, (D1-1) the alkali metal silicate has a composition represented by the following general formula (d1):

$$x(M_2O)\cdot y(SiO_2)\cdot z(Me_mO_n)\cdot w(H_2O) \qquad (d1)$$

wherein M represents one or two or more combinations selected from the group consisting of alkali metals, Me is one or two or more elements selected from of the Groups II, III, IV, and VIII of the periodic table of elements, y/x = 0.5 or more and 2.6 or less, z/x = 0.01 or more and 10 or less, w = 0 or more and 20 or less, and n/m = 0.5 or more and 20 or less.

Examples of the alkali metal as M in general formula (d1) include Na, K and others. These may be used singly or for example, $Na_2O$ and $K_2O$ may be mixed, to constitute the $M_2O$ component. Examples of Me include Mg, Ca, Zn, Y, Ti, Zr, and Fe. These are not particularly limited, but from the viewpoint of resources and safety, are preferably Mg and Ca. These may be used singly or in mixture of two or more thereof, and, for example, MgO, CaO, or the like may be mixed to constitute a MemOn component.

In general formula (d1), y/x is 0.5 or less and 2.6 or less, and preferably 1.5 or more and 2.2 or less. When y/x exceeds 2.6, the ion exchange capacity becomes low. Also, in general formula (d-1), when z/x exceeds 1.0, the ion exchange capacity becomes low. x, y and z are not particularly limited as long as they have such a relationship as given by y/x ratio and z/x ratio described above. When x ($M_2O$) is, for example, x' ($Na_2O$)·x'' ($K_2O$) as described above, x is x' + x". Such a relationship also applies to z in the case of a z($Me_mO_n$) component composed of two or more components. n/m represents the number of oxygen ions coordinated to the elements, and is substantially selected from values of 0.5, 1.0, 1.5 and 2.0.

The alkali metal silicate represented by general formula (d-1) preferably has an ion exchange capacity of 100 $CaCO_3$ mg/g or more, preferably 200 $CaCO_3$ mg/g or more, 600 $CaCO_3$ mg/g or less and is one of the substances having an ion capture capacity in the present invention.

(D1-2) The aluminosilicate may be either crystalline or amorphous, but is preferably a crystalline aluminosilicate, as a divalent compound having a high metal ion-exchange capture capacity. The crystalline aluminosilicate is generally referred to as zeolite, and examples thereof include those represented by the following general formula (d2):

$$a'(M_2O)\cdot Al_2O_3\cdot b'(SiO_2)\cdot w(H_2O) \qquad (d2)$$

wherein M represents an alkali metal atom, a', b', and w each represent the molar number of each component, generally, $0.7 \leq a' \leq 1.5$ and $0.8 \leq b' < 6$, and w is an optional positive number. Among others, those represented by the following general formula (d3) are preferred:

$$Na_2O\cdot Al_2O_3\cdot n(SiO_2)\cdot w(H_2O) \qquad (d3)$$

wherein n represents a number of 1.8 or more and 3.0 or less, and w represents a number of 1 or more and 6 or less.

As a crystalline aluminosilicate (zeolite), a synthetic zeolite having an average primary particle diameter of 0.1 to 10 μm, typified by type A-, type X-and type P-zeolite, is preferably used. The zeolite may be blended as agglomerated dried zeolite particles obtained by drying a zeolite powder and/or a zeolite slurry.

Examples of the counter ion that forms the salt of (D1-3) the tripolyphosphate include an ion selected from a sodium ion and a potassium ion. That is, examples of (D1-3) include compounds selected from sodium tripolyphosphate and potassium tripolyphosphate.

[(D2) Metal ion chelating agent as organic compound]

Examples of (D2) the metal ion chelating agent as an organic compound include one or more organic compounds selected from (D2-1) divalent or higher and tetravalent or lower carboxylic acids having 4 or more and 12 or less carbons and containing no amino group or a salt thereof, (D2-2) divalent or higher and tetravalent or lower carboxylic acids having 4 or more and 10 or less carbons and containing an amino group or a salt thereof, and (D2-3) organic compounds having a phosphonic acid group or a salt thereof in the molecule.

Specific examples of the divalent or higher and tetravalent or lower carboxylic acid having 4 or more and 12 or less carbons and containing no amino group or a salt thereof as component (D2-1) include one or more carboxylic acids selected from citric acid, tartaric acid, succinic acid, succinic acid, and salts thereof.

Specific examples of the divalent or higher and tetravalent or lower carboxylic acid having 4 or more and 12 or less carbons and containing an amino group or a salt thereof as component (D2-2) include one or more carboxylic acids selected from nitrilotriacetic acid, ethylenediaminetetraacetic acid, 3-hydroxy-2,2'-iminodisuccinic acid, diethylenetriaminepentaacetic acid, hydroxymethylethylenediaminetriacetic acid, and salts thereof.

Examples of (D2-3) the organic compound having a phosphonic acid group or a salt thereof in the molecule include organic phosphonic acid derivatives such as ethane-1,1-diphosphonic acid, ethane-l,1,2-triphosphonic acid, 1-hydroxyethylidene-1,1-diphosphonic acid, ethanehydroxy-1,1,2-triphosphonic acid, ethane-1,2-dicarboxy-1,2-diphosphonic acid, methane hydroxyphosphonic acid, nitrilotrimethylene phosphonic acid, and ethylenediamine tetrakis(methylenephosphonic acid).

The salts of component (D2-1) to component (D2-3) are preferably salts of an alkali metal such as Na or K and salts of an alkanolamine having 2 to 6 carbons such as monoethanolamine, diethanolamine or triethanolamine, from the viewpoint of versatility.

The method for treating a textile product, for example, a washing method of the present invention, especially

when employed for treatment with water containing a hardness component, can provide a more excellent effect of retaining the water absorbency of the textile product. Thus, it is possible to reduce the content of component (D) in treating liquid 1. From this viewpoint, in the method for treating a textile product, for example, a washing method of the present invention, the mass ratio of the content of component (D) to the content of component (A) in treating liquid 1, component (D)/component (A) is preferably 1 or less, more preferably 0.5 or less, further preferably 0.3 or less, furthermore preferably 0.1% by mass or less, furthermore preferably 0.05% by mass or less, and 0 or more. Component (D)/component (A) may be 0.

Treating liquid 1 is preferably a detergent liquid. Accordingly, step 1 may be a step of washing a textile product by contacting detergent liquid 1 obtained by mixing water and component (A) with the textile product. The matters mentioned above can be appropriately applied also to this step.

In treating liquid 1, the content of component (B) is preferably 20 mg/kg or less, more preferably 10 mg/kg or less, further preferably 5 mg/kg or less, furthermore preferably 4 mg/kg or less. Treating liquid 1 may not contain component (B).

<Step 2>

Step 2 is a step of treating the textile product treated in step 1 by contacting the textile product with treating liquid 2 obtained by mixing water and a finishing agent composition for textile products. By performing step 2, it is possible to impart a texture to the textile product while the water absorbency inherently possessed by the textile product is retained. The texture herein refers to, but not limited to, feelings such as softness, fluffy feeling, elasticity, smoothness, pliability, tension feeling, and slimy feeling, for example. Imparting a texture to a textile product means that, for example, in the treating method, for example, the washing method of the present invention, when a textile product obtained by drying the textile product obtained in step 2 and a textile product obtained by drying the textile product obtained in step 1 are compared, the above-mentioned texture is felt in the textile product obtained by drying the textile product in step 2. The finishing agent composition for textile products contains a finishing base for textile products.

In step 2, water and the finishing agent composition for textile products are mixed to obtain treating liquid 2. The water used in step 2 may be referred to as water 2 hereinafter. Water 2 may be the same as water 1 used in step 1 above or may be different. Water 1, together with the textile product obtained in step 1, may be carried over to and contained in water 2 of step 2.

The finishing agent composition for textile products to be used in step 2 means the finishing agent composition for textile products.

In step 2, water 2 and the finishing agent composition for textile products constitute a mixture. This mixture is treating liquid 2.

The content of an active component contained in the finishing agent composition for textile products in treating liquid 2 is, from the viewpoint of imparting a texture to the textile product, preferably 5 mg/kg or more, more preferably 10 mg/kg or more, further preferably 15 mg/kg or more, furthermore preferably 20 mg/kg or more, and preferably 1000 mg/kg or less, more preferably 700 mg/kg or less, further preferably 500 mg/kg or less, furthermore preferably 300 mg/kg or less, furthermore preferably 100 mg/kg or less. The active component contained in the finishing agent composition for textile products refers to a specific component, removal of which from the finishing agent composition for textile products lowers the texture imparting effect to a textile product. The active component can be component (B) in the case of being the finishing agent composition for textile products containing a cationic surfactant, a silicone compound, a fatty alcohol, a fatty acid ester, a fat and oil, a fatty acid or a salt thereof, as component (B), without limitation.

In the case where the finishing agent composition for textile products in treating liquid 2 contains one or more cationic surfactants (B1) as component (B), the content of component (B1) in treating liquid 2 is, from the viewpoint of imparting a texture to the textile product, preferably 5 mg/kg or more, more preferably 10 mg/kg or more, further preferably 15 mg/kg or more, furthermore preferably 20 mg/kg or more, furthermore preferably 25 mg/kg or more, and preferably 1000 mg/kg or less, more preferably 700 mg/kg or less, further preferably 500 mg/kg or less, furthermore preferably 300 mg/kg or less, furthermore preferably 100 mg/kg or less.

In the case where the finishing agent composition for textile products in treating liquid 2 contains (B1) one or more cationic surfactants and (B2) one or more silicone compounds as component (B), the mass ratio of component (B2)/component (B1) is preferably 0.01 or more, more preferably 0.05 or more, and preferably 0.1 or less, more preferably 1 or less.

In the case where the finishing agent composition for textile products in treating liquid 2 contains one or more components selected from (B3) fatty alcohols, (B4) fatty acid esters, (B5) fats and oils, and (B6) fatty acids or salts thereof as component (B), the content of each component is, from the viewpoint of imparting a texture to the textile product, preferably 5 mg/kg or more, more preferably 10 mg/kg or more, further preferably 15 mg/kg or more, furthermore preferably 20 mg/kg or more, furthermore preferably 25 mg/kg or more, and preferably 1000 mg/kg or less, more preferably 700 mg/kg or less, further preferably 500 mg/kg or less, furthermore preferably 400 mg/kg or less, furthermore preferably

300 mg/kg or less.

In the case where the finishing agent composition for textile products in treating liquid 2 contains one or more components selected from (B1) one or more cationic surfactants, and (B3) fatty alcohols, (B4) fatty acid esters, (B5) fats and oils, and (B6) fatty acids or salts thereof as component (B), the mass ratio of [one or more components selected from component (B3) to component (B6)]/[component (B1)] is preferably 0.01 or more, more preferably 0.05 or more, further preferably 0.1 or more, and preferably 100 or less, more preferably 50 or less, further preferably 30 or less.

Treating liquid 2 can contain components other than component (B), for example, optional components such as component (C) and component (D) mentioned for treating liquid 1. These optional components can be selected in consideration of finishing treatment to be conducted in step 2. These optional components may be blended in the finishing agent composition for textile products or may be blended separately from the composition.

Treating liquid 2 may contain component (A) carried over from treating liquid 1. That is, for example, component (A) in treating liquid 1 remaining in the textile product after step 1 or component (A) derived from treating liquid 1 adhering to the textile product after step 1 may be released into treating liquid 2 and carried over into treating liquid 2. From the viewpoint of retaining water absorbency possessed by a textile product and the viewpoint of modifying a function of the finishing base for textile products contained in the finishing agent composition for textile products, the content of component (A) in treating liquid 2 is preferably 500 mg/kg or less, more preferably 300 mg/kg or less, further preferably 100 mg/kg or less, furthermore preferably 50 mg/kg or less, furthermore preferably 30 mg/kg or less, and preferably 0 mg/kg or more, more preferably more than 0 mg/kg. Alternatively treating liquid 2 may not contain component (A).

In step 2, the temperature of treating liquid 2 may be, for example, a temperature of preferably 0°C or more, more preferably 3°C or more, further preferably 5°C or more, and preferably 40°C or less, more preferably 35°C or less.

The pH of treating liquid 2 at 25°C is, from the viewpoint of improving the effect of imparting a texture to the textile product, preferably 4 or more, more preferably 5 or more, and preferably 10.5 or less, more preferably 10 or less, further preferably 9 or less, furthermore preferably 8.5 or less, furthermore preferably 8.0 or less. The pH can be measured in accordance with the method for measuring the pH.

The value of the bath ratio expressed as the ratio of the volume of water 2 or treating liquid 2 (liter) to the mass of the textile product (kg), that is, volume of water or treating liquid 2 (liter)/mass of textile product (kg) may be referred to as bath ratio 2 hereinafter. In step 2, the finishing agent composition for textile products can impart a texture to the textile product. Bath ratio 2 is, from the viewpoint of being capable of inhibiting decrease in the effect of the finishing agent composition for textile products to impart a texture to the textile product, preferably 2 or more, more preferably 3 or more, further preferably 4 or more, furthermore preferably 5 or more, and preferably 45 or less, more preferably 40 or less, further preferably 30 or less, furthermore preferably 20 or less. The mass of the textile product in bath ratio 2 is the mass of the textile product used first in step 1. In the case where the textile product after step 1 is in a moistened state, the amount of treating liquid 1 in step 1 taken into the textile product is not included.

The contact time between the textile product and treating liquid 2 in step 2 is, from the viewpoint of being capable of further enhancing the effect of imparting a texture to the textile product, preferably 10 seconds or more, more preferably 30 seconds or more, further preferably 1 minute or more, more preferably 2 minutes or more, further preferably 3 minutes or more, and preferably 30 minutes or less, further preferably 20 minutes or less, furthermore preferably 10 minutes or less, more preferably 5 minutes or less.

A rotary washing machine can be used for treatment of the textile product in step 2. Specific examples of the rotary washing machine include drum washing machines, pulsator washing machines, or agitator washing machines. As these rotary washing machines, those commercially available for household use may be each used. In respect of enabling further reduction in the amount of water to be used for one round of treatment, for example, one round of washing, drum washing machines, which have rapidly spread in recent years, can reduce the amount of water especially in washing. In respect of being capable of further benefiting from effects of the present invention, a drum washing machine is preferably used to conduct step 2.

In step 2, leaving treating liquid 2 obtained from water 2 and the finishing agent composition for textile products to stand after being contacted with the textile product can impart a texture to the textile product. Leaving to stand means a state in which the textile product, water 2, and the finishing agent composition for textile products are contacted with one another and then left to stand. A stirring operation such as shaking and manual agitation may be conducted before or after the above leaving-to-stand, or furthermore, in the course of the leaving-to-stand.

<Optional steps>

[Spin-drying step]

Between step 1 and step 2, for example, before the textile product obtained in step 1 is subject to step 2, a spin-drying step of spin-drying the textile product treated in step 1 can be conducted. Alternatively, after step 2, a spin-drying step of spin-drying the textile product treated in step 2 can be conducted. Hereinafter, the spin-drying step to be

conducted between step 1 and step 2 may be referred to as spin-drying step 1. The spin-drying step to be conducted after step 2 may be referred to as spin-drying step 2. Spin-drying steps 1 and 2 can be each conducted a plurality of times.

Spin-drying step 1 refers to a step of reducing the amount of treating liquid 1 coexisting with the textile product. By performing spin-drying step 1, it is possible to reduce the amount of the anionic surfactant to be carried over together with the textile product to step 2. It is known that the anionic surfactant reduces the effect of the active component contained in the finishing agent composition for textile products to impart a texture to the textile product. Conducting spin-drying step 1 is preferable in respect of further enhancing the effect of imparting a texture to the textile product in step 2. When the mass of the textile product subjected to step 1 is taken as 100 parts by mass, the textile product removed from treating liquid 1 in step 1 may contain treating liquid 1 of 90 parts by mass or more and of the order of 200 parts by mass. Spin-drying step 1 means a step of reducing the mass of treating liquid 1 contained in the textile product to 10 parts by mass or more and 70 parts by mass or less, when the mass of the textile product before subjected to step 1 is taken as 100 parts by mass. An example of spin-drying step 1 is a method including reducing the amount of treating liquid 1 by applying gravity, centrifugal force, or pressure from the textile product, from a state in which the textile product and treating liquid 1 coexist.

Spin-drying step 2 is a step of reducing the amount of treating liquid 2 existing with the textile product obtained in step 2. By conducting spin-drying step 2, the drying time mentioned below is shortened and the textile product becomes suitable for wearing. When the mass of the textile product before subjected to step 1 is taken as 100 parts by mass, spin-drying step 2 means a step of reducing the mass of treating liquid 2 to 10 parts by mass or more and 70 parts by mass or less. An example of spin-drying step 2 is a method including reducing the amount of treating liquid 2 coexisting with the textile product by applying gravity, centrifugal force, or pressure from the textile product, from a state in which the textile product and treating liquid 2 coexist.

[Rinsing step]

A rinsing step may be conducted between step 1 and step 2. The rinsing step in the present invention refers to a step of reducing the amount of the anionic surfactant present in the textile product obtained in step 1. An example of the rinsing step is a method including contacting the textile product treated in step 1 with fresh water. In the rinsing step, the value of the bath ratio expressed as the ratio of the volume of water (liter) to the mass of the textile product (kg) before subjected to step 1, that is, volume of water (liter)/mass of textile product (kg) may be referred to as bath ratio 3 hereinafter. Bath ratio 3 is, from the viewpoint of being capable of inhibiting decrease in the effect of the finishing agent composition for textile products to impart a texture to the textile product in step 2, preferably 2 or more, more preferably 3 or more, further preferably 4 or more, furthermore preferably 5 or more, and preferably 45 or less, more preferably 40 or less, further preferably 30 or less, furthermore preferably 20 or less. Water used in the rinsing step may be the same as or different from water 1 and water 2. The rinsing step can be conducted a plurality of times.

[Drying step]

A drying step for drying the textile product may be conducted between step 1 and step 2 or after step 2.

The drying step is a step of reducing the amount of treating liquid 1 coexisting with the textile product obtained in step 1 or of treating liquid 2 coexisting with the textile product obtained in step 2. Specifically, the drying step refers to causing the mass of the textile product after dried to be 90 parts by mass or more and 110 parts by mass or less, when the mass of the textile product before subjected to step 1 is taken as 100 parts by mass. In step 1 and step 2 described above or an optional step, fibers may fall off from the textile product, and the mass of the textile product after the drying step may apparently decrease below the mass of the textile product before subjected to step 1. Thus, the mass of the textile product after drying is taken as the above range. Drying may be either of natural drying or heating drying. The drying step can be conducted a plurality of times.

<Water absorbency retaining agent for textile products>

The present invention provides a water absorbency retaining agent for textile products containing (A) an internal olefin sulfonate having 17 or more and 24 or less carbons [component (A)]. The water absorbency retaining agent for textile products of the present invention is an agent that imparts an effect of retaining water absorbency inherently possessed by a textile product to the textile product. Treating a textile product with the water absorbency retaining agent for textile products of the present invention enables the water absorbency to be retained even when the textile product is further treated with a treatment agent such as a softener. For example, when treatment with a softener by a user is contemplated, use of the water absorbency retaining agent for textile products of the present invention enables selection of a softener in preference of fragrance and other physical properties to water absorbency. The water absorbency retaining agent for textile products of the present invention is preferably used before treatment with a finishing agent

composition for textile products, for example, a softener.

The water absorbency retaining agent for textile products of the present invention may be composed of component (A). The water absorbency retaining agent for textile products of the present invention may also be a water absorbency retaining agent composition for textile products containing component (A) and other components. Examples of the water absorbency retaining agent composition for textile products include those containing component (A) and water. In the case of the water absorbency retaining agent composition for textile products, the content of component (A) can be 3% by mass or more and 60% by mass or less.

<Function modifier for a finishing base for textile products>

The present invention provides a function modifier for a finishing base for textile products containing (A) an internal olefin sulfonate having 17 or more and 24 or less carbons [component (A)]. The function modifier for a finishing base for textile products of the present invention is an agent that imparts an effect of inhibiting changes in water absorbency inherently possessed by a textile product to the finishing base for textile products while retaining the texture imparting effect of the finishing base for textile products to the textile product. Treating a textile product with the function modifier for a finishing base for textile products of the present invention enables the water absorbency to be retained even when the textile product is further treated with a finishing base for textile products. For example, when treatment with a finishing base for textile products by a user is contemplated, use of the function modifier for a finishing base for textile products of the present invention enables selection of a finishing base for textile products in preference of fragrance and other physical properties such as the texture of the textile product to water absorbency. The function modifier for a finishing base for textile products of the present invention is preferably used before treatment with a finishing base for textile products.

The function modifier for a finishing base for textile products of the present invention may be composed of component (A). The function modifier for a finishing base for textile products of the present invention may be a function modifier composition for a finishing base for textile products containing component (A) and other components. Examples of the function modifier for a finishing base for textile products include those containing component (A) and water. In the case of the function modifier composition for a finishing base for textile products, the content of component (A) can be 3% by mass or more and 60% by mass or less.

<Embodiments of the present invention>

More specific embodiments of the present invention will be illustrated as follows. The matters mentioned as for the method for treating a textile product, the method for producing a textile product, the method for retaining water absorbency of a textile product, the water absorbency retaining agent for textile products, the method for modifying a function of a finishing base for textile products, and the function modifier for a finishing base for textile products of the present invention can be appropriately applied to the following aspects.

<1> A method for treating a textile product, for example, a method for washing a textile product, including the following step 1 and step 2:

step 1: a step of treating the textile product by contacting the textile product with treating liquid 1 obtained by mixing water and (A) an internal olefin sulfonate having 17 or more and 24 or less carbons [hereinafter, referred to as component (A)]; and

step 2: a step of treating the textile product treated in step 1 by contacting the textile product with treating liquid 2 obtained by mixing water and a finishing agent composition for textile products.

<2> The method for treating a textile product according to <1>, wherein component (A) is an internal olefin sulfonate obtained by using, as raw material, internal olefins including an internal olefin having 17 or more and 24 or less carbons with a double bond located at position 5 or higher.

<3> The method for treating a textile product according to <2>, wherein component (A) is an internal olefin sulfonate obtained by using, as raw material, internal olefins containing 10% by mass or more and 60% by mass or less of the internal olefin having 17 or more and 24 or less carbons with a double bond located at position 5 or higher.

<4> The method for treating a textile product according to <3>, wherein the content of an internal olefin sulfonate including the sulfonate group located at position 5 or higher in component (A) is 10.5% by mass or more, preferably 11.0% by mass or more, more preferably 12.0% by mass or more, further preferably 12.5% by mass or more, furthermore preferably 13.0% by mass or more, furthermore preferably 13.5% by mass or more, and 55% by mass or less, preferably 50% by mass or less, more preferably 45% by mass or less, further preferably 40% by mass or less, furthermore preferably 35% by mass or less, furthermore preferably 30% by mass or less.

<5> The method for treating a textile product according to <1> or <2>, wherein component (A) includes an internal olefin sulfonate having 17 or more and 24 or less carbons with the sulfonate group located at position 2 or higher and position 4 or lower (IO-1S) and an internal olefin sulfonate having 17 or more and 24 or less carbons with the sulfonate group located at position 5 or higher (IO-2S), and the mass ratio of (IO-1S) to (IO-2S), (IO-1S)/(IO-2S) is 0.6 or more and 6 or less.
<6> The method for treating a textile product according to <5>, wherein, component (A) has a mass ratio of the content of (IO-1S) to the content of (IO-2S), (IO-1S)/(IO-2S) of 0.65 or more, preferably 0.70 or more, more preferably 0.75 or more, further preferably 0.8 or more, furthermore preferably 1.0 or more, furthermore preferably 1.2 or more, furthermore preferably 1.4 or more, furthermore preferably 1.6 or more, furthermore preferably 2.0 or more, furthermore preferably 2.5 or more, furthermore preferably 3.0 or more, furthermore preferably 3.5 or more, furthermore preferably 4.0 or more, and 5.9 or less, preferably 5.8 or less, more preferably 5.7 or less, further preferably 5.6 or less, furthermore preferably 5.5 or less, furthermore preferably 5.4 or less, furthermore preferably 5.3 or less, furthermore preferably 5.2 or less, furthermore preferably 5.1 or less, furthermore preferably 5.0 or less, furthermore preferably 4.9 or less, furthermore preferably 4.8 or less, furthermore preferably 4.7 or less.
<7> The method for treating a textile product according to any of <1> to <6>, wherein the number of carbons of component (A) is preferably 18 or more, and preferably 22 or less, more preferably 20 or less, further preferably 19 or less.
<8> The method for treating a textile product according to any of <1> to <7>, wherein the content of an olefin sulfonate including the sulfonate group located at position 1 in component (A) is 10% by mass or less, more preferably 7% by mass or less, further preferably 5% by mass or less, furthermore preferably 3% by mass or less, and 0.01% by mass or more, preferably 0.02% by mass or more, more preferably 0.03% by mass or more, further preferably 0.04% by mass or more in component (A).
<9> The method for treating a textile product according to any of <1> to <8>, wherein the finishing agent composition for textile products contains a finishing base for textile products as component (B).
<10> The method for treating a textile product according to any of <1> to <9>, wherein component (B) as the finishing base for textile products is one or more components selected from (B1) cationic surfactants having a hydrocarbon group having 16 or more and 24 or less, (B2) silicone compounds, (B3) fatty alcohols, (B4) fatty acid esters, (B5) fats and oils, and (B6) fatty acids or salts thereof.
<11> The method for treating a textile product according to any of <1> to <10>, wherein the textile product is a textile product containing cotton.
<12> A method for producing a textile product, including the following step 1 and step 2:

step 1: a step of treating the textile product by contacting the textile product with treating liquid 1 obtained by mixing water and (A) an internal olefin sulfonate having 17 or more and 24 or less carbons [hereinafter, referred to as component (A)]; and
step 2: a step of treating the textile product treated in step 1 by contacting the textile product with treating liquid 2 obtained by mixing water and a finishing agent composition for textile products.

<13> The method for producing a textile product according to <12>, wherein component (A) is an internal olefin sulfonate obtained by using, as raw material, internal olefins including an internal olefin having 17 or more and 24 or less carbons with a double bond located at position 5 or higher.
<14> The method for producing a textile product according to <13>, wherein component (A) is an internal olefin sulfonate obtained by using, as raw material, internal olefins containing 10% by mass or more and 60% by mass or less of the internal olefin having 17 or more and 24 or less carbons with a double bond located at position 5 or higher.
<15> The method for producing a textile product according to <14>, wherein the content of an internal olefin sulfonate including the sulfonate group located at position 5 or higher in component (A) is 10.5% by mass or more, preferably 11.0% by mass or more, more preferably 12.0% by mass or more, further preferably 12.5% by mass or more, furthermore preferably 13.0% by mass or more, furthermore preferably 13.5% by mass or more, and 55% by mass or less, preferably 50% by mass or less, more preferably 45% by mass or less, further preferably 40% by mass or less, furthermore preferably 35% by mass or less, furthermore preferably 30% by mass or less.
<16> The method for producing a textile product according to <12> or <13>, wherein component (A) includes an internal olefin sulfonate having 17 or more and 24 or less carbons with the sulfonate group located at position 2 or higher and position 4 or lower (IO-1S) and an internal olefin sulfonate having 17 or more and 24 or less carbons with the sulfonate group located at position 5 or higher (IO-2S), and the mass ratio of (IO-1S) to (IO-2S), (IO-1S)/(IO-2S) is 0.6 or more and 6 or less.
<17> The method for producing a textile product according to <16>, wherein, component (A) has a mass ratio of the content of (IO-1S) to the content of (IO-2S), (IO-1S)/(IO-2S) of 0.65 or more, preferably 0.70 or more, more preferably 0.75 or more, further preferably 0.8 or more, furthermore preferably 1.0 or more, furthermore preferably

1.2 or more, furthermore preferably 1.4 or more, furthermore preferably 1.6 or more, furthermore preferably 2.0 or more, furthermore preferably 2.5 or more, furthermore preferably 3.0 or more, furthermore preferably 3.5 or more, furthermore preferably 4.0 or more, and 5.9 or less, preferably 5.8 or less, more preferably 5.7 or less, further preferably 5.6 or less, furthermore preferably 5.5 or less, furthermore preferably 5.4 or less, furthermore preferably 5.3 or less, furthermore preferably 5.2 or less, furthermore preferably 5.1 or less, furthermore preferably 5.0 or less, furthermore preferably 4.9 or less, furthermore preferably 4.8 or less, furthermore preferably 4.7 or less.

<18> The method for producing a textile product according to any of <12> to <17>, wherein the number of carbons of component (A) is preferably 18 or more, and preferably 22 or less, more preferably 20 or less, further preferably 19 or less.

<19> The method for producing a textile product according to any of <12> to <18>, wherein the content of an olefin sulfonate including the sulfonate group located at position 1 in component (A) is 10% by mass or less, more preferably 7% by mass or less, further preferably 5% by mass or less, furthermore preferably 3% by mass or less, and 0.01% by mass or more, preferably 0.02% by mass or more, more preferably 0.03% by mass or more, further preferably 0.04% by mass or more in component (A).

<20> The method for producing a textile product according to any of <1> to <19>, wherein the finishing agent composition for textile products contains a finishing base for textile products as component (B).

<21> The method for producing a textile product according to any of <1> to <20>, wherein component (B) as the finishing base for textile products is one or more components selected from (B1) cationic surfactants having a hydrocarbon group having 16 or more and 24 or less carbons, (B2) silicone compounds, (B3) fatty alcohols, (B4) fatty acid esters, (B5) fats and oils, and (B6) fatty acids or salts thereof.

<22> The method for producing a textile product according to any of <1> to <21>, wherein the textile product is a textile product containing cotton.

<23> A method for retaining water absorbency of a textile product, wherein, in treating the textile product by steps including the following step 1 and step 2, (A) an internal olefin sulfonate having 17 or more and 24 or less carbons is employed as an anionic surfactant of step 1:

step 1: a step of treating the textile product by contacting the textile product with treating liquid 1 obtained by mixing water and the anionic surfactant; and

step 2: a step of treating the textile product treated in step 1 by contacting the textile product with treating liquid 2 obtained by mixing water and a finishing agent composition for textile products.

<24> The method for retaining water absorbency of a textile product according to <23>, wherein component (A) is an internal olefin sulfonate obtained by using, as raw material, internal olefins including an internal olefin having 17 or more and 24 or less carbons with a double bond located at position 5 or higher.

<25> The method for retaining water absorbency of a textile product according to <24>, wherein component (A) is an internal olefin sulfonate obtained by using, as raw material, internal olefins containing 10% by mass or more and 60% by mass or less of the internal olefin having 17 or more and 24 or less carbons with a double bond located at position 5 or higher.

<26> The method for retaining water absorbency of a textile product according to <25>, wherein the content of an internal olefin sulfonate including the sulfonate group located at position 5 or higher in component (A) is 10.5% by mass or more, preferably 11.0% by mass or more, more preferably 12.0% by mass or more, further preferably 12.5% by mass or more, furthermore preferably 13.0% by mass or more, furthermore preferably 13.5% by mass or more, and 55% by mass or less, preferably 50% by mass or less, more preferably 45% by mass or less, further preferably 40% by mass or less, furthermore preferably 35% by mass or less, furthermore preferably 30% by mass or less.

<27> The method for retaining water absorbency of a textile product according to <23> or <24>, wherein component (A) includes an internal olefin sulfonate having 17 or more and 24 or less carbons with the sulfonate group located at position 2 or higher and position 4 or lower (IO-1S) and an internal olefin sulfonate having 17 or more and 24 or less carbons with the sulfonate group located at position 5 or higher (IO-2S), and the mass ratio of (IO-1S) to (IO-2S), (IO-1S)/(IO-2S) is 0.6 or more and 6 or less.

<28> The method for retaining water absorbency of a textile product according to <27>, wherein, component (A) has a mass ratio of the content of (IO-1S) to the content of (IO-2S), (IO-1S)/(IO-2S) of 0.65 or more, preferably 0.70 or more, more preferably 0.75 or more, further preferably 0.8 or more, furthermore preferably 1.0 or more, furthermore preferably 1.2 or more, furthermore preferably 1.4 or more, furthermore preferably 1.6 or more, furthermore preferably 2.0 or more, furthermore preferably 2.5 or more, furthermore preferably 3.0 or more, furthermore preferably 3.5 or more, furthermore preferably 4.0 or more, and 5.9 or less, preferably 5.8 or less, more preferably 5.7 or less, further preferably 5.6 or less, furthermore preferably 5.5 or less, furthermore preferably 5.4 or less, furthermore preferably 5.3 or less, furthermore preferably 5.2 or less, furthermore preferably 5.1 or less, furthermore preferably 5.0 or less, furthermore preferably 4.9 or less, furthermore preferably 4.8 or less, furthermore preferably 4.7 or less.

<29> The method for retaining water absorbency of a textile product according to any of <23> to <28>, wherein the number of carbons of component (A) is preferably 18 or more, and preferably 22 or less, more preferably 20 or less, further preferably 19 or less.

<30> The method for retaining water absorbency of a textile product according to any of <23> to <29>, wherein the content of an olefin sulfonate including the sulfonate group located at position 1 in component (A) is 10% by mass or less, more preferably 7% by mass or less, further preferably 5% by mass or less, furthermore preferably 3% by mass or less, and 0.01% by mass or more, preferably 0.02% by mass or more, more preferably 0.03% by mass or more, further preferably 0.04% by mass or more in component (A).

<31> The method for retaining water absorbency of a textile product according to any of <23> to <30>, wherein the finishing agent composition for textile products contains a finishing base for textile products as component (B).

<32> The method for retaining water absorbency of a textile product according to any of <23> to <231>, wherein component (B) as the finishing base for textile products is one or more components selected from (B1) cationic surfactants having a hydrocarbon group having 16 or more and 24 or less carbons, (B2) silicone compounds, (B3) fatty alcohols, (B4) fatty acid esters, (B5) fats and oils, and (B6) fatty acids or salts thereof.

<33> The method for retaining water absorbency of a textile product according to any of <23> to <32>, wherein the textile product is a textile product containing cotton.

<34> A water absorbency retaining agent for textile products, containing (A) an internal olefin sulfonate having 17 or more and 24 or less carbons.

<35> The water absorbency retaining agent for textile products according to <34>, wherein component (A) is an internal olefin sulfonate obtained by using, as raw material, internal olefins including an internal olefin having 17 or more and 24 or less carbons with a double bond located at position 5 or higher.

<36> The water absorbency retaining agent for textile products according to <35>, wherein component (A) is an internal olefin sulfonate obtained by using, as raw material, internal olefins containing 10% by mass or more and 60% by mass or less of the internal olefin having 17 or more and 24 or less carbons with a double bond located at position 5 or higher.

<37> The water absorbency retaining agent for textile products according to <36>, wherein the content of an internal olefin sulfonate including the sulfonate group located at position 5 or higher in component (A) is preferably 10.5% by mass or more, more preferably 11.0% by mass or more, further preferably 12.0% by mass or more, furthermore preferably 12.5% by mass or more, furthermore preferably 13.0% by mass or more, furthermore preferably 13.5% by mass or more, and preferably 55% by mass or less, more preferably 50% by mass or less, further preferably 45% by mass or less, furthermore preferably 40% by mass or less, furthermore preferably 35% by mass or less, furthermore preferably 30% by mass or less.

<38> The water absorbency retaining agent for textile products according to <34> or <35>, wherein component (A) includes an internal olefin sulfonate having 17 or more and 24 or less carbons with the sulfonate group located at position 2 or higher and position 4 or lower (IO-1S) and an internal olefin sulfonate having 17 or more and 24 or less carbons with the sulfonate group located at position 5 or higher (IO-2S), and the mass ratio of (IO-1S) to (IO-2S), (IO-1S)/(IO-2S) is 0.6 or more and 6 or less.

<39> The water absorbency retaining agent for textile products according to <38>, wherein, component (A) has a mass ratio of the content of (IO-1S) to the content of (IO-2S), (IO-1S)/(IO-2S) of 0.65 or more, preferably 0.70 or more, more preferably 0.75 or more, further preferably 0.8 or more, furthermore preferably 1.0 or more, furthermore preferably 1.2 or more, furthermore preferably 1.4 or more, furthermore preferably 1.6 or more, furthermore preferably 2.0 or more, furthermore preferably 2.5 or more, furthermore preferably 3.0 or more, furthermore preferably 3.5 or more, furthermore preferably 4.0 or more, and 5.9 or less, preferably 5.8 or less, more preferably 5.7 or less, further preferably 5.6 or less, furthermore preferably 5.5 or less, furthermore preferably 5.4 or less, furthermore preferably 5.3 or less, furthermore preferably 5.2 or less, furthermore preferably 5.1 or less, furthermore preferably 5.0 or less, furthermore preferably 4.9 or less, furthermore preferably 4.8 or less, furthermore preferably 4.7 or less.

<40> The water absorbency retaining agent for textile products according to any of <34> to <39>, wherein the number of carbons of component (A) is preferably 18 or more, and preferably 22 or less, more preferably 20 or less, further preferably 19 or less.

<41> The water absorbency retaining agent for textile products according to any of <34> to <40>, wherein the content of an olefin sulfonate including the sulfonate group located at position 1 in component (A) is 10% by mass or less, more preferably 7% by mass or less, further preferably 5% by mass or less, furthermore preferably 3% by mass or less, and 0.01% by mass or more, preferably 0.02% by mass or more, more preferably 0.03% by mass or more, further preferably 0.04% by mass or more in component (A).

<42> A method for modifying a function of a finishing base for textile products, wherein, in treating a textile product by steps including the following step 1 and step 2, (A) an internal olefin sulfonate having 17 or more and 24 or less carbons is employed as an anionic surfactant of step 1:

step 1: a step of treating the textile product by contacting the textile product with treating liquid 1 obtained by mixing water and the anionic surfactant; and

step 2: a step of treating the textile product treated in step 1 by contacting the textile product with treating liquid 2 obtained by mixing water and a finishing agent composition for textile products containing a finishing base for textile products.

<43> The method for modifying a function of a finishing base for textile products according to <42>, wherein component (A) is an internal olefin sulfonate obtained by using, as raw material, internal olefins including an internal olefin having 17 or more and 24 or less carbons with a double bond located at position 5 or higher.

<44> The method for modifying a function of a finishing base for textile products according to <43>, wherein component (A) is an internal olefin sulfonate obtained by using, as raw material, internal olefins containing 10% by mass or more and 60% by mass or less of the internal olefin having 17 or more and 24 or less carbons with a double bond located at position 5 or higher.

<45> The method for modifying a function of a finishing base for textile products according to <44>, wherein the content of the internal olefin sulfonate including an sulfonate group located at position 5 or higher in component (A) is preferably 10.5% by mass or more, more preferably 11.0% by mass or more, further preferably 12.0% by mass or more, furthermore preferably 12.5% by mass or more, furthermore preferably 13.0% by mass or more, furthermore preferably 13.5% by mass or more, and preferably 55% by mass or less, more preferably 50% by mass or less, further preferably 45% by mass or less, furthermore preferably 40% by mass or less, furthermore preferably 35% by mass or less, furthermore preferably 30% by mass or less.

<46> The method for modifying a function of a finishing base for textile products according to <42> or <43>, wherein component (A) includes an internal olefin sulfonate having 17 or more and 24 or less carbons with the sulfonate group located at position 2 or higher and position 4 or lower (IO-1S) and an internal olefin sulfonate having 17 or more and 24 or less carbons with the sulfonate group located at position 5 or higher (IO-2S), and the mass ratio of (IO-1S) to (IO-2S), (IO-1S)/(IO-2S) is 0.6 or more and 6 or less.

<47> The method for modifying a function of a finishing base for textile products according to <46>, wherein, component (A) has a mass ratio of the content of (IO-1S) to the content of (IO-2S), (IO-1S)/(IO-2S) of 0.65 or more, preferably 0.70 or more, more preferably 0.75 or more, further preferably 0.8 or more, furthermore preferably 1.0 or more, furthermore preferably 1.2 or more, furthermore preferably 1.4 or more, furthermore preferably 1.6 or more, furthermore preferably 2.0 or more, furthermore preferably 2.5 or more, furthermore preferably 3.0 or more, furthermore preferably 3.5 or more, furthermore preferably 4.0 or more, and 5.9 or less, preferably 5.8 or less, more preferably 5.7 or less, further preferably 5.6 or less, furthermore preferably 5.5 or less, furthermore preferably 5.4 or less, furthermore preferably 5.3 or less, furthermore preferably 5.2 or less, furthermore preferably 5.1 or less, furthermore preferably 5.0 or less, furthermore preferably 4.9 or less, furthermore preferably 4.8 or less, furthermore preferably 4.7 or less.

<48> The method for modifying a function of a finishing base for textile products according to any of <42> to <47>, wherein the number of carbons of component (A) is preferably 18 or more, and preferably 22 or less, more preferably 20 or less, further preferably 19 or less.

<49> The method for modifying a function of a finishing base for textile products according to any of <42> to <48>, wherein the content of an olefin sulfonate including the sulfonate group located at position 1 in component (A) is 10% by mass or less, more preferably 7% by mass or less, further preferably 5% by mass or less, furthermore preferably 3% by mass or less, and 0.01% by mass or more, preferably 0.02% by mass or more, more preferably 0.03% by mass or more, further preferably 0.04% by mass or more in component (A).

<50> The method for modifying a function of a finishing base for textile products according to any of <42> to <49>, wherein the finishing agent composition for textile products contains a finishing base for textile products as component (B).

<51> The method for modifying a function of a finishing base for textile products according to any of <42> to <50>, wherein component (B) as the finishing base for textile products is one or more components selected from (B1) cationic surfactants having hydrocarbon group having 16 or more and 24 or less carbons, (B2) silicone compounds, (B3) fatty alcohols, (B4) fatty acid esters, (B5) fats and oils, and (B6) fatty acids or salts thereof.

<52> The method for modifying a function of a finishing base for textile products according to any of <42> to <51>, wherein the textile product is a textile product containing cotton.

<53> A function modifier for a finishing base for textile products, containing (A) an internal olefin sulfonate having 17 or more and 24 or less carbons.

<54> The function modifier for a finishing base for textile products according to <53>, wherein component (A) is an internal olefin sulfonate obtained by using, as raw material, internal olefins including an internal olefin having 17 or more and 24 or less carbons with a double bond located at position 5 or higher.

<55> The function modifier for a finishing base for textile products according to <54>, wherein component (A) is an

internal olefin sulfonate obtained by using, as raw material, internal olefins containing 10% by mass or more and 60% by mass or less of the internal olefin having 17 or more and 24 or less carbons with a double bond located at position 5 or higher.

<56> The function modifier for a finishing base for textile products according to <55>, wherein the content of an internal olefin sulfonate including the sulfonate group located at position 5 or higher in component (A) is preferably 10.5% by mass or more, more preferably 11.0% by mass or more, further preferably 12.0% by mass or more, furthermore preferably 12.5% by mass or more, furthermore preferably 13.0% by mass or more, furthermore preferably 13.5% by mass or more, and preferably 55% by mass or less, more preferably 50% by mass or less, further preferably 45% by mass or less, furthermore preferably 40% by mass or less, furthermore preferably 35% by mass or less, furthermore preferably 30% by mass or less.

<57> The function modifier for a finishing base for textile products according to <53> or <54>, wherein component (A) includes an internal olefin sulfonate having 17 or more and 24 or less carbons with the sulfonate group located at position 2 or higher and position 4 or lower (IO-1S) and an internal olefin sulfonate having 17 or more and 24 or less carbons with the sulfonate group located at position 5 or higher (IO-2S), and the mass ratio of (IO-1S) to (IO-2S), (IO-1S)/(IO-2S) is 0.6 or more and 6 or less.

<58> The function modifier for a finishing base for textile products according to <57>, wherein, component (A) has a mass ratio of the content of (IO-1S) to the content of (IO-2S), (IO-1S)/(IO-2S) of 0.65 or more, preferably 0.70 or more, more preferably 0.75 or more, further preferably 0.8 or more, furthermore preferably 1.0 or more, furthermore preferably 1.2 or more, furthermore preferably 1.4 or more, furthermore preferably 1.6 or more, furthermore preferably 2.0 or more, furthermore preferably 2.5 or more, furthermore preferably 3.0 or more, furthermore preferably 3.5 or more, furthermore preferably 4.0 or more, and 5.9 or less, preferably 5.8 or less, more preferably 5.7 or less, further preferably 5.6 or less, furthermore preferably 5.5 or less, furthermore preferably 5.4 or less, furthermore preferably 5.3 or less, furthermore preferably 5.2 or less, furthermore preferably 5.1 or less, furthermore preferably 5.0 or less, furthermore preferably 4.9 or less, furthermore preferably 4.8 or less, furthermore preferably 4.7 or less.

<59> The function modifier for a finishing base for textile products according to any of <53> to <58>, wherein the number of carbons of component (A) is preferably 18 or more, and preferably 22 or less, more preferably 20 or less, further preferably 19 or less.

<60> The function modifier for a finishing base for textile products according to any of <53> to <59>, wherein the content of an olefin sulfonate including the sulfonate group located at position 1 in component (A) is 10% by mass or less, more preferably 7% by mass or less, further preferably 5% by mass or less, furthermore preferably 3% by mass or less, and 0.01% by mass or more, preferably 0.02% by mass or more, more preferably 0.03% by mass or more, further preferably 0.04% by mass or more in component (A).

## Examples

[Synthesis of components (A) and (a'-1)]

(1) Synthesis internal olefins A to C (Production Examples A to C)

Internal olefins A to C to be used as the raw material for component (A) were synthesized as follows.

Into a flask equipped with a stirring device, 7000 g (25.9 mol) of 1-octadecanol (product name: KALCOL 8098 manufactured by Kao Corporation) and 700 g (10% by mass based on the raw material alcohol) of γ-alumina (STREM Chemicals, Inc.) as a solid acid catalyst were introduced, and allowed to react at 280°C under stirring for a different reaction time for each of Production Examples A to C while passing nitrogen (7000 mL/min) through the flask. The resulting crude internal olefin was transferred to a distillation flask and subjected to distillation at 148-158°C/0.5 mmHg to obtain each of internal olefins A to C having 18 carbons at an olefin purity of 100%. The double bond distribution of each of the obtained internal olefins is shown in Table 1.

(2) Internal olefin D having 16 carbons for (a'-1)

As internal olefin D having 16 carbons, an internal olefin obtained by using the method described in Production Example C of JP-A 2014-76988 was used. The double bond distribution of internal olefin D is shown in Table 1.

[Table 1]

| | | Internal olefin | | | |
|---|---|---|---|---|---|
| | | A | B | C | D |
| Number of carbons of olefin | | 18 | 18 | 18 | 16 |
| Composition in olefin (% by mass) | Linear olefin | 99.8 | 99.3 | 99.0 | 99.5 |
| | Branched olefin | 0.2 | 0.7 | 1.0 | 0.5 |
| Double bond distribution in linear olefin (% by mass) | Position 1 | 1.6 | 0.9 | 0.3 | 0.5 |
| | Position 2 | 41.7 | 25.0 | 13.3 | 30.1 |
| | Position 3 | 29.3 | 21.9 | 12.6 | 25.5 |
| | Position 4 | 15.7 | 19.0 | 13.9 | 18.9 |
| | Position 5 | 6.3 | 13.6 | 14.8 | 11.0 |
| | Position 6 | 3.9 | 8.6 | 13.7 | 7.0 |
| | Position 7 | 1.1 | 5.6 | 12.6 | 3.5 |
| | Position 8 | 0.2 | 2.7 | 9.4 | 3.5 |
| | Position 9 | 0.2 | 2.7 | 9.4 | 0.0 |
| | Position 5 or higher | 11.7 | 33.2 | 59.9 | 25.0 |

The double bond distribution of each of the internal olefins was measured by gas chromatography (hereinafter abbreviated as GC). Specifically, the internal olefin was allowed to react with dimethyl disulfide to form its dithiolated derivative, and each component was then separated by GC. Consequently, the double bond distribution in the internal olefin was determined from each of the resulting peak areas. As for the olefins having 18 carbons, the internal olefin having a double bond at position 8 and the internal olefin having a double bond at position 9 cannot be distinguished from each other in structure but distinguished when they are sulfonated. Thus, the value obtained by dividing the amount of the internal olefin having a double bond at position 8 by 2 is conveniently shown in each column. Similarly, for the olefins having 16 carbons, the value obtained by dividing the amount of the internal olefin having a double bond at position 7 by 2 is conveniently shown in each column.

The devices and the analysis conditions used for the measurement are as follows: GC apparatus: "HP6890" (manufactured by Hewlett-Packard Company); column: "Ultra-Alloy-1 HT Capillary Column" (30 m × 250 μm × 0.15 μm, manufactured by Frontier Laboratories, Ltd.); detector (hydrogen flame ionization detector (FID)); injection temperature: 300°C; detector temperature: 350°C; and He flow rate: 4.6 mL/min.

(3) Synthesis of (a-1), (a-2), (a-3), and (a-1')

The internal olefins A to D were subjected to sulfonation reaction using a thin film-type sulfonation reactor equipped with an external jacket by passing sulfur trioxide gas through the reactor while cooling water at 20°C was passed through the external jacket. In the sulfonation reaction, the molar ratio, $SO_3$/internal olefin, was set to 1.09. The sulfonated product obtained was added to an alkali aqueous solution prepared using sodium hydroxide in a molar amount of 1.5 times the theoretical acid value, followed by neutralization at 30°C for 1 hour under stirring. The neutralized product was heated in an autoclave at 160°C for 1 hour for hydrolysis, to obtain a crude product of each internal sodium olefin sulfonate. 300 g of the crude product was transferred to a separation funnel, and 300 mL of ethanol was added thereto. Thereafter, 300 mL of petroleum ether was added per time, to extract and remove oil-soluble impurities. At this time, inorganic compounds (mainly containing sodium sulfate) precipitated at the interface of oil and water due to the addition of ethanol were also separated and removed from the water phase by the oil-water separation operation. The extraction and removal operation was repeated 3 times. The water phase was evaporated to dryness, to obtain an internal sodium olefin sulfonate as follows. Herein, the internal olefin sulfonate obtained by using internal olefin A as the raw material was used as (a-1), the internal olefin sulfonate obtained by using internal olefin B as the raw material was used as (a-2), the internal olefin sulfonate obtained by using internal olefin C as the raw material was used as (a-3), and the internal olefin sulfonate obtained by using internal olefin D as the raw material was used as (a'-1). Additionally, (a-1) and (a-2) were mixed to obtain (a-4), and (a-2) and (a-3) were mixed to obtain (a-5), as described below.

The proportion of the content of the internal olefin sulfonate with the sulfonate group attached thereto was

determined by high performance liquid chromatography/mass spectrometer (HPLC-MS). Specifically, identification was conducted by separating the hydroxy form having a sulfonic acid group attached thereto by high performance liquid chromatography (HPLC) and subjecting the hydroxy form to a mass spectrometer (MS). Each proportion was determined from the resulting HPLC-MS peak areas. Herein, each proportion determined from the peak area of the HAS form was calculated as proportion by mass.

The devices and the conditions used for the measurement are as follows: HPLC apparatus: "LC-20ASXR" (manufactured by Shimadzu Corporation); column: "ODS Hypersil (R)" (4.6 × 250 mm, particle size: 3 μm, manufactured by Thermo Fisher Scientific K.K.); sample preparation (1000 times diluted with methanol); eluent A (10 mM ammonium acetate-added water); eluent B (10 mM ammonium acetate-added, methacrylonitrile/water = 95/5 (v/v) solution); gradient (0 minute (A/B = 60/40) → 15.1 to 20 minutes (30/70) → 20.1 to 30 minutes (60/40); MS apparatus "LCMS-2020" (manufactured by Shimadzu Corporation); ESI detection (negative ion detection, m/z: 349.15 (component (A) having 18 carbons), 321.10 (component (D) having 16 carbons); column temperature (40°C); flow rate (0.5 mL/min); and injection volume (5 μL).

The distribution of the positions of the carbon to which the sulfonate group each of (a-1), (a-2), (a-3), (a-4), (a-5), and (a'-1) obtained is attached is shown in Table 2.

[Table 2]

| | | | Component (A) | | | | | Component |
|---|---|---|---|---|---|---|---|---|
| | | | (a-1) | (a-2) | (a-3) | (a-4) | (a-5) | (a'-1) |
| Number of carbons of hydrocarbon group | | | 18 | 18 | 18 | 18 | 18 | 16 |
| Sulfonate group distribution (% by mass) | Position 1 | | 1.6 | 1.4 | 0.6 | 1.6 | 0.8 | 1.5 |
| | (IO-1S) | Position 2 | 31.5 | 22.1 | 12.8 | 29.4 | 14.8 | 24.1 |
| | | Position 3 | 25.1 | 17.2 | 10.7 | 23.4 | 12.1 | 19.9 |
| | | Position 4 | 24.7 | 21.8 | 16.6 | 24.1 | 17.7 | 24.6 |
| | (IO-2S) | Position 5 or higher | 17.1 | 37.5 | 59.3 | 21.5 | 54.6 | 29.9 |
| Content of IO-1S (% by mass) | | | 81.3 | 61.1 | 40.1 | 76.9 | 44.6 | 68.6 |
| (IO-1S)/(IO-2S) (mass ratio) | | | 4.8 | 1.6 | 0.68 | 3.6 | 0.82 | 2.3 |

[Synthesis of component (B1)]

(1) Synthesis of component (b1-1): cationic surfactant

195 g (0.71 mol) of mixed fatty acid 1 (mass ratio of palmitic acid/stearic acid/oleic acid/linoleic acid: 30/30/35/5, average molecular weight: 275) and 54.4 g (0.37 mol) of triethanolamine were mixed and allowed to react at 180 to 185°C (under normal pressure) for 3 hours. Then, the pressure was reduced to 200 mmHg, and aging was further conducted for 3 hours. Thereafter, the pressure was returned to normal level with nitrogen, and the reaction product was cooled to 100°C to obtain 392 g of a dehydrated condensate. The acid value of the condensate obtained (in compliance with JIS K0070) was 0.7 mgKOH/g, and the total amine value thereof (in compliance with JIS K2501) was 196 mgKOH/g. Then, the temperature of 392 g of this dehydrated condensate was adjusted to 70 to 75°C, and on the basis of the amine value of the dehydrated condensate, dimethyl sulfate in an amount corresponding to 0.98 equivalents relative to the amine equivalent of the dehydrated condensate was added dropwise over 2.5 hours. After the dropwise addition was completed, the dehydrated condensate was further kept at 50 to 55°C for 3 hours to obtain a reaction product containing the intended compound (b1-1) of 75% by mass. In Examples, a quaternary ammonium salt in the reaction product was used as component (b1-1).

(2) Synthesis of component (b1-2): cationic surfactant

195 g (0.71 mol) of mixed fatty acid 2 (mass ratio of palmitic acid/stearic acid/oleic acid/linoleic acid: 35/60/4/1, average molecular weight: 274) and 54.4 g (0.37 mol) of triethanolamine were mixed and allowed to react at 180 to 185°C (under normal pressure) for 3 hours. Then, the pressure was reduced to 200 mmHg, and aging was further conducted for 3 hours. Thereafter, the pressure was returned to normal level with nitrogen, and the reactant was cooled to 100°C to obtain 394 g of a dehydrated condensate. The acid value of the condensate obtained (in compliance with

JIS K0070) was 1.0 mgKOH/g, and the total amine value thereof (in compliance with JIS K2501) was 197 mgKOH/g. Then, the temperature of 394 g of this dehydrated condensate was adjusted to 70 to 75°C, and on the basis of the amine value of the dehydrated condensate, dimethyl sulfate in an amount corresponding to 0.98 equivalents relative to the amine equivalent of the dehydrated condensate was added dropwise over 2.5 hours. After the dropwise addition was completed, the dehydrated condensate was further kept at 50 to 55°C for 3 hours to obtain a reaction product containing the intended compound (b1-2) of 76% by mass. In Examples, a quaternary ammonium salt in the reaction product was used as component (b1-2).

(3) Synthesis of component (b1-3): cationic surfactant

200 g (0.71 mol) of mixed fatty acid 3 (mass ratio of palmitic acid/stearic acid/oleic acid/linoleic acid: 2/3/80/15, average molecular weight: 281) and 54.4 g (0.37 mol) of triethanolamine were mixed and allowed to react at 180 to 185°C (under normal pressure) for 3 hours. Then, the pressure was reduced to 200 mmHg, and aging was further conducted for 3 hours. Thereafter, the pressure was returned to normal level with nitrogen, and the reactant was cooled to 100°C to obtain 397 g of a dehydrated condensate. The acid value of the condensate obtained (in compliance with JIS K0070) was 1.5 mgKOH/g, and the total amine value thereof (in compliance with JIS K2501) was 190 mgKOH/g. Then, the temperature of 397 g of this dehydrated condensate was adjusted to 70 to 75°C, and on the basis of the amine value of the dehydrated condensate, dimethyl sulfate in an amount corresponding to 0.98 equivalents relative to the amine equivalent of the dehydrated condensate was added dropwise over 2.5 hours. After the dropwise addition was completed, the dehydrated condensate was further kept at 50 to 55°C for 3 hours to obtain a reaction product containing the intended compound (b1-3) of 80% by mass. In Examples, a quaternary ammonium salt in the reaction product was used as component (b1-3).

<Components to be blended>

[Component (A)]

(a-1): Sodium internal olefin sulfonate obtained from internal olefin A
The mass ratio of the hydroxy form (sodium hydroxyalkane sulfonate)/the olefin form (sodium olefin sulfonate) in the sodium internal olefin sulfonate: 82/18
(a-2): Sodium internal olefin sulfonate obtained from internal olefin B
The mass ratio of the hydroxy form (sodium hydroxyalkane sulfonate)/the olefin form (sodium olefin sulfonate) in the sodium internal olefin sulfonate: 83/17
(a-3): Sodium internal olefin sulfonate obtained from internal olefin C
The mass ratio of the hydroxy form (sodium hydroxyalkane sulfonate)/the olefin form (sodium olefin sulfonate) in the sodium internal olefin sulfonate: 83/17
(a-4): Internal olefin sulfonate (a-4) in Table 2
(a-5): Internal olefin sulfonate (a-5) in Table 2

[component (A')] (comparative component of component A)

(a'-1): Sodium internal olefin sulfonate obtained from internal olefin D
The mass ratio of the hydroxy form (sodium hydroxyalkane sulfonate)/the olefin form (sodium olefin sulfonate) in the sodium internal olefin sulfonate: 84/16
(a'-2): Sodium alkylbenzene sulfonate (Neopelex G-25, manufactured by Kao Corporation)
(a'-3): Component (a-1) described in Example of JP-A 2011-63784: a compound obtained by adding 9 mol of ethylene oxide per 1 mol of a primary alcohol having 10 to 14 carbons to react therebetween, then adding 2 mol of propylene oxide to react therewith, and then allowing 9 mol of ethylene oxide to react therewith.

[Component (B)]

(b1-1): Quaternary ammonium salt obtained in "(1) Synthesis of component (b1-1): cationic surfactant" mentioned

above
(b1-2): Quaternary ammonium salt obtained in "(2) Synthesis of component (b1-2): cationic surfactant" mentioned above
(b1-3): Quaternary ammonium salt obtained in "(3) Synthesis of component (b1-3): cationic surfactant" mentioned above
(b2-1): Dimethyl polysiloxane (manufactured by Shin-Etsu Chemical Co., Ltd.)
(b2-2): Polyether-modified silicone (manufactured by Shin-Etsu Chemical Co., Ltd.)
(b3-1): Fatty alcohol (mixed alcohol of lauryl alcohol/myristyl alcohol/palmityl alcohol = 60/30/10 (mass ratio))
(b4-1): Monoglyceride stearate (EXCEL 84 manufactured by Kao Corporation)
(b6-1): Fatty acid sodium (mixed fatty acid sodium of sodium myristate/sodium palmitate/sodium stearate = 5/30/65 (mass ratio))

[Water]

Water: Tap water (3.5°dH, calculated by the method for measuring German hardness of water, 20°C)
Exemplary treatment of textile products of the present invention and comparative textile products is shown below. Treatment of a textile product also can be read as washing of a textile product.

[Examples 1 to 6, Comparative Examples 1 to 2, and Reference Examples 1 to 2]

The softness, water absorbency, and detergency in the case of treatment of a textile product with treating liquid 1 and treating liquid 2 shown in Table 3 were evaluated by the following methods.

<Evaluation of softness and water absorbency>

(1a) Preparation of textile product for evaluation of softness and water absorbency

As a textile product, knitted cotton cloth (un-mercerized knitted cotton (not mercerized), cotton 100%, manufactured by Shikisensha Co., Ltd.) was used.
1.7 kg of the knitted cotton cloth was washed cumulatively twice with a standard course of a fully automatic washing machine (NA-F702 P manufactured by Matsushita Electric Industrial Co., Ltd.) (in washing, 4.7 g of the surfactant Emulgen 108 (manufactured by Kao Corporation, used as was); the amount of water: 47 L; washing for 9 minutes, rinsing twice, and spin-drying for 3 minutes) followed by cumulatively washing three times with water only (the amount of water: 47 L; washing for 9 minutes, rinsing twice, and spin-drying for 3 minutes), and dried under an environment of 23°C and 45% RH for 24 hours. This provided a textile product for evaluation of softness and water absorbency.

(2a) Treatment of textile product for evaluation

[Step 1]

Into an electric bucket type washing machine (model number "N-BK2"), manufactured by Matsushita Electric Industrial Co., Ltd., 4.0 L of municipal water was poured. Subsequently, a 5% by mass aqueous solution of component (A) or component (A') described in Table 3 was introduced thereinto, and treating liquid 1 was prepared such that the concentration of component (A) or component (A') in treating liquid 1 reached the concentration of Table 2. Then, stirring was conducted for 1 minute to prepare treating liquid 1 as a mixture of municipal water and component (A) or component (A'). The concentration of component (A) or component (A') in treating liquid 1 is shown in Table 3. The results of the pH of treating liquid 1 measured in accordance with the pH measurement method are described in Table 3. The temperature of treating liquid 1 is shown in Table 3. 4 pieces of the textile product for evaluation (70 g/piece) obtained in (1a) described above were introduced into the washing machine and washed for 10 minutes by stirring.

[Spin-drying step 1]

The 4 pieces of the textile product for evaluation subjected to step 1 described above were spin-dried for 1 minute using a two-compartment washing machine manufactured by Hitachi, Ltd. (model number: "PS-H35L"). As a result of determination of the mass of the textile product for evaluation after the spin-drying, the mass of the mixture after spin-drying was 70 parts by mass in any of Examples or Comparative Examples, when the mass of the textile product for evaluation before subjected to step 1 was taken as 100 parts by mass.

[Rinsing step 1]

Into an electric bucket type washing machine (model number "N-BK2"), manufactured by Matsushita Electric Industrial Co., Ltd., 4.0 L of municipal water was poured. Subsequently, the 4 pieces of the textile product for evaluation that finished the spin-drying step 1 were introduced, and then, 6.0 L of municipal water was poured into the bucket type washing machine. Additionally, after spin-drying with a two-compartment washing machine manufactured by Hitachi, Ltd., a textile product for evaluation was introduced, and a rinsing step was conducted for 3 minutes. Thereafter, spin-drying was conducted for 1 minute using the two-compartment washing machine.

[Spin-drying step 2]

The 4 pieces of the textile product for evaluation that had finished rinsing step 1 described above were spin-dried for 1 minute using a two-compartment washing machine manufactured by Hitachi, Ltd. (model number: "PS-H35L"). As a result of determination of the mass of the textile product for evaluation after the spin-drying, the mass of the mixture after spin-drying was 69 parts by mass in any of Examples or Comparative Examples, when the mass of the textile product for evaluation before subjected to step 1 was taken as 100 parts by mass.

[Drying step 1]

The textile product for evaluation that had finished spin-drying step 2 was hung on a laundry pole and left to stand under conditions of 20°C and 43% RH for 12 hours to dryness.

[Step 2]

The mass of the textile product for evaluation that had finished drying step 1 was determined (70 g/piece). Into an electric bucket type washing machine (model number "N-BK2"), manufactured by Matsushita Electric Industrial Co., Ltd., 4.0 L of municipal water was poured. Subsequently, a 5% by mass aqueous dispersion of component (B) described in Table 3 was introduced thereto and stirring was conducted for 1 minute. Treating liquid 2 as a mixture of component (B) and water was obtained such that the concentration of component (B) in treating liquid 2 reached the concentration of Table 3. The temperature and pH of treating liquid 2 were 20°C and 7.3, respectively. The 4 pieces of textile product for evaluation (70 g/piece) that had finished drying step 1 were introduced into the washing machine and stirred for 3 minutes to bring the textile product for evaluation into contact with treating liquid 2. The 5% by mass aqueous dispersion of component (B) is a finishing agent composition for textile products that contains component (B) of 5% by mass, as a finishing base for textile products.

[Spin-drying step 3]

The 4 pieces of the textile product for evaluation that had finished step 2 described above were spin-dried for 1 minute using a two-compartment washing machine manufactured by Hitachi, Ltd. (model number: "PS-H35L"). As a result of determination of the mass of the textile product for evaluation after the spin-drying, the mass of the mixture after spin-drying was 69 parts by mass in any of Examples or Comparative Examples, when the mass of the textile product for evaluation before subjected to step 2 was taken as 100 parts by mass.

[Drying step 2]

The textile product for evaluation that had finished spin-drying step 2 was hung on a laundry pole and left to stand under conditions of 20°C and 43% RH for 12 hours to dryness.
The treatment on the textile product for evaluation included step 1, spin-drying step 1, rinsing step 1, spin-drying step 2, drying step 1, step 2, spin-drying step 3, and drying step 2 described above as one cycle. The textile product for evaluation after a total of 3 cycles were finished was used to evaluate softness and water absorbency as shown below. The results are shown in Table.
The mass of the textile product for evaluation was a value determined under an environment of 20°C and 43% RH in any of the steps.

(3a) Softness evaluation

Expert panelists compared and evaluated the softness of the textile product for evaluation treated in Reference Example 1 and of the textile products for evaluation each treated in Examples 1 to 6, Comparative Example 1 or

Comparative Example 2.

(4a) Water absorbency evaluation

2 pieces were randomly chosen out of 4 pieces of knitted cotton cloth as the textile product for evaluation. From each piece of the knitted cotton cloth, one piece of 25 cm in length and 2.5 cm in width was cut out each from the 2 pieces of the knitted cotton cloth. A point 2 cm from the end of a short side along the long side direction was marked with a black aqueous marker. The point marked was set as 0 cm, and a mark was placed every 1 cm along the long side direction up to 20 cm. The fabric piece was placed with the direction in which the mark of 0 cm was placed downward, the opposite short side upward, and the long side direction perpendicular to the water surface. Then, 2 L of tap water at 25°C was placed into a plastic beaker (capacity: 2 liters), and the short side of the fabric piece (lower end) was immersed in the water until the water surface reached the mark of 0 cm. The time at which the water surface reached 0 cm was taken as 0 minute, and the height of water in 15 minutes was measured. The top end to which the black mark blurred to cause the black ink to develop by chromatography was taken as the height of water. The average value of the two fabric pieces is shown in Table 3. The piece having the higher water absorption height exhibits better water absorbency. The height of water of each of Examples and Comparative Examples was designated as "water absorption height 1". Additionally, the height of water of Reference Example 1 (reference 2), which is a treatment method in which the piece is treated with no anionic surfactant and with no finishing agent composition for textile products, was designated "water absorption height 2". The value of water absorption height 1/water absorption height 2, as the ratio of water absorption height 1 of Examples 1 to 6 and Comparative Example 1 or Comparative Example 2 to water absorption height 2, was calculated. Water absorption height 1/water absorption height 2 corresponds to water absorbency 1/water absorbency 2. A value of 0.72 or more is acceptable, and a value closer to 1 is preferable.

(5a) Detergency evaluation

A washing procedure was conducted in the same manner using a textile product for evaluation obtained in the following (5a-1) instead of the textile product for evaluation obtained in (1a) in the above treatment in (2a) textile product for evaluation.

(5a-1) Preparation of textile product for detergency evaluation

0.5 mL of a model artificial sebum stained liquid having the following composition was applied to a plain-woven portion of the knitted cotton cloth obtained (1a) in a circle having a diameter of 2 cm and dried under an environment of 23°C and 45% RH for 24 hours. This provided a textile product for detergency evaluation. *Composition of model artificial sebum stained liquid: lauric acid: 0.4% by mass, myristic acid: 3.1% by mass, pentadecanoic acid: 2.3% by mass, palmitic acid: 6.2% by mass, heptadecanoic acid: 0.4% by mass, stearic acid: 1.6% by mass, oleic acid: 7.8% by mass, triolein: 13.0% by mass, n-hexadecyl palmitate: 2.2% by mass, squalene: 6.5% by mass, egg white lecithin liquid crystal product: 1.9% by mass, Kanuma red clay: 8.1% by mass, carbon black: 0.01% by mass, and water: balance (100% by mass in total)

[Table 3]

| | | | | | Example | | | | | | Comparative Example | | Reference Example | |
|---|---|---|---|---|---|---|---|---|---|---|---|---|---|---|
| | | | | | 1 | 2 | 3 | 4 | 5 | 6 | 1 (reference 1) | 2 | 1 (reference 2) | 2 |
| Step 1 | Treating liquid 1 | Composition (mg/kg) | (A) | (a-1) | 170 | | | | | | | | | |
| | | | | (a-2) | | 170 | | 170 | | | | | | |
| | | | | (a-3) | | | 170 | | | | | | | |
| | | | | (a-4) | | | | | 170 | | | | | |
| | | | | (a-5) | | | | | | 170 | | | | |
| | | | (A') | (a'-1) | | | | | | | 170 | | | |
| | | | | (a'-2) | | | | | | | | 35 | | 35 |
| | | | | (a'-3) | | | | | | | | 135 | | 135 |
| | | | | Water | Balance | Balance | Balance | Balance | Balance | Balance | Balance | Balance | Balance (water only) | Balance |
| | | pH of treating liquid 1 (25°C) | | | 7.0 | 7.0 | 7.0 | 7.0 | 7.0 | 7.0 | 7.0 | 7.0 | 7.0 | 7.0 |
| | | Temperature of treating liquid 1 (°C) | | | 25 | 25 | 25 | 25 | 25 | 25 | 25 | 25 | 25 | 25 |
| Step 2 | Treating liquid 2 | Composition (mg/kg) | (B) | (b1-1) | 40 | 40 | 40 | 40 | 40 | 40 | 40 | 40 | | |
| | | | | (b2-1) | 5 | 5 | 5 | | 5 | 5 | 5 | 5 | | |
| | | | | (b2-2) | | | | 5 | | | | | | |
| | | | | Water | Balance | Balance | Balance | Balance | Balance | Balance | Balance | Balance | Balance (water only) | Balance (water only) |
| | | pH of treating liquid 2 (25°C) | | | 7.0 | 7.0 | 7.0 | 7.0 | 7.0 | 7.0 | 7.0 | 7.0 | 7.0 | 7.0 |
| | | Temperature of treating liquid 2 (°C) | | | 25 | 25 | 25 | 25 | 25 | 25 | 25 | 25 | 25 | 25 |

(continued)

| | | Example | | | | | | Comparative Example | | Reference Example | |
|---|---|---|---|---|---|---|---|---|---|---|---|
| | | 1 | 2 | 3 | 4 | 5 | 6 | 1 (reference 1) | 2 | 1 (reference 2) | 2 |
| Evaluation | Water absorbency (water absorption height in 15 minutes, cm) | 8.6 | 8.2 | 7.6 | 8.3 | 8.4 | 8.0 | 4.8 | 4.2 | 8.6 | 8.3 |
| | Water absorption height 1/water absorption height 2 (water absorption height 2: reference 2) | 1.0 | 0.95 | 0.88 | 0.97 | 0.97 | 0.91 | 0.56 | 0.49 | 1.0 | 0.97 |

The water absorption height of the knitted cotton cloth each of Examples 1 to 6, which was washed with component (A) of the present invention and then treated by being contacted with the finishing agent composition for textile products, was higher than that of the cotton knitted cotton cloth of Comparative Example 1 (reference 1), which was washed with an internal olefin sulfonate not corresponding to component (A) and then contacted with and treated with the finishing agent composition for textile products. Water absorption height 1/water absorption height 2, as the ratio of water absorption height 1 of Examples 1 to 6 to water absorption height 2 of the knitted cotton cloth treated in Reference Example 1 (reference 2), which is a treatment method in which the fabric is treated with no anionic surfactant and with no finishing agent composition for textile products, was 0.72 or more and close to 1 in each of the Examples, each providing substantially comparable water absorbency. The water absorbency of the knitted cotton cloth that was washed with Comparative Example 2 in Table 1 and then contacted with and treated with the finishing agent composition for textile products was comparable with that of Comparative Example 1 (reference 1).

As for the softness felt when the knitted cotton cloth was touched by hand, the knitted cotton cloth treated in each of the Examples 1 to 6, Comparative Example 1 or Comparative Example 2 was softer than the cotton knitted cotton cloth treated in Reference Example 1.

As for the detergency, as a result of visual observation of the degree of staining by the model sebum attached to the knitted cotton cloth after treatment in Examples 1 to 6 or Comparative Example 1, the degree of staining was lower in each of Examples 1 to 6 and Comparative Example 1 than that of the knitted cotton cloth after treated in Reference Example 1, and the stains attached to the knitted cotton cloth had been washed off.

[Examples 7 to 9 and Reference Examples 3 to 6]

The water absorbency in the case of treatment of textile products with treating liquid 1 and treating liquid 2 shown in Table 4 was evaluated by the following method.

<Water absorbency evaluation>

(1b) Preparation of textile product for evaluation

As a textile product, cotton towels (Takei Towel Co., Ltd., TW-220, including a plain-woven portion, cotton 100%) were used.

24 cotton towels were washed cumulatively twice with a standard course of a fully automatic washing machine (NA-F702P manufactured by Matsushita Electric Industrial Co., Ltd.) (in washing, 4.7 g of the surfactant Emulgen 108 (manufactured by Kao Corporation, used as was); the amount of water: 47 L; washing for 9 minutes, rinsing twice, and spin-drying for 3 minutes) followed by cumulatively washing three times with water only (the amount of water: 47 L; washing for 9 minutes, rinsing twice, and spin-drying for 3 minutes), and dried under an environment of 23°C and 45% RH for 24 hours. This provided the textile product for water absorbency evaluation.

(2b) Washing of textile product for evaluation

[Step 1]

Into an electric bucket type washing machine (model number "N-BK2"), manufactured by Matsushita Electric Industrial Co., Ltd., 4.0 L of municipal water was poured. Subsequently, a 5% by mass aqueous solution of component (A) or component (A') described in Table 4 was introduced thereinto, and treating liquid 1 was prepared such that the concentration of component (A) or component (A') in treating liquid 1 reached the concentration of Table 4. Then, stirring was conducted for 1 minute to prepare treating liquid 1 as a mixture of municipal water and component (A) or component (A'). The concentration of component (A) or component (A') in treating liquid 1 is shown in Table 4. The results of the pH of treating liquid 1 measured in accordance with the pH measurement method are described in Table 4. The temperature of treating liquid 1 is shown in Table 4. The 2 pieces of the textile product for evaluation obtained in (1b) (140 g) were introduced into the washing machine and washed for 10 minutes by stirring.

[Spin-drying step 1]

The 2 pieces of the textile product for evaluation that had finished step 1 described above (140 g) were spin-dried for 1 minute using a two-compartment washing machine manufactured by Hitachi, Ltd. (model number: "PS-H35L"). As a result of determination of the mass of the textile product for evaluation after the spin-drying, the mass of the mixture after spin-drying was 80 parts by mass in any of Examples or Comparative Examples, when the mass of the textile product for evaluation before subjected to step 1 was taken as 100 parts by mass.

[Rinsing step 1]

Into an electric bucket type washing machine (model number "N-BK2"), manufactured by Matsushita Electric Industrial Co., Ltd., 4.0 L of municipal water was poured. Subsequently, the 2 pieces of the textile product for evaluation that finished the spin-drying step 1 were introduced, and then, 6.0 L of municipal water was poured into the bucket type washing machine. Additionally, after spin-drying with a two-compartment washing machine manufactured by Hitachi, Ltd., a textile product for evaluation was introduced, and a rinsing step was conducted for 3 minutes. Thereafter, spin-drying was conducted for 1 minute using the two-compartment washing machine.

[Spin-drying step 2]

The 2 pieces of the textile product for evaluation that had finished rinsing step 1 were spin-dried for 1 minute using a two-compartment washing machine manufactured by Hitachi, Ltd. (model number: "PS-H35L"). As a result of determination of the mass of the textile product for evaluation after the spin-drying, the mass of the mixture after spin-drying was 83 parts by mass in any of Examples or Comparative Examples, when the mass of the textile product for evaluation before subjected to step 1 was taken as 100 parts by mass.

[Drying step 1]

The textile product for evaluation that had finished spin-drying step 2 was hung on a laundry pole and left to stand under conditions of 20°C and 43% RH for 12 hours to dryness.

[Step 2]

The mass of the textile product for evaluation that had finished drying step 1 was determined (139 g/piece). The mass of the textile product for evaluation decreased in some degrees. It is conceived that some of fibers constituting the towels fell off in the course of the step 1 to drying step 1. Into an electric bucket type washing machine (model number "N-BK2"), manufactured by Matsushita Electric Industrial Co., Ltd., 4.0 L of municipal water was poured. Subsequently, a 5% by mass aqueous dispersion of component (B) described in Table 4 was introduced thereto and stirring was conducted for 1 minute. Treating liquid 2 as a mixture of component (B) and water was obtained such that the concentration of component (B) in treating liquid 2 reached the concentration of Table 4. The temperature and pH of treating liquid 2 were 20°C and 7.3, respectively. The 2 pieces of textile product for evaluation (139 g/piece) that had finished drying step 1 were introduced into the washing machine and stirred for 3 minutes to bring the 2 pieces of the textile product for evaluation into contact with treating liquid 2. The 5% by mass aqueous dispersion of component (B) is a finishing agent composition for textile products that contains component (B) of 5% by mass, as a finishing base for textile products.

[Spin-drying step 3]

The 2 pieces of the textile product for evaluation that had finished step 2 described above were spin-dried for 1 minute using a two-compartment washing machine manufactured by Hitachi, Ltd. (model number: "PS-H35L"). As a result of determination of the mass of the textile product for evaluation after the spin-drying, the mass of the mixture after spin-drying was 82 parts by mass in any of Examples or Comparative Examples, when the mass of the textile product for evaluation before subjected to step 2 was taken as 100 parts by mass.

[Drying step 2]

The textile product for evaluation obtained in spin-drying step 2 was hung on a laundry pole and left to stand under conditions of 20°C and 43% RH for 12 hours to dryness.

The treatment on the textile product for evaluation included step 1, spin-drying step 1, rinsing step 1, spin-drying step 2, drying step 1, step 2, spin-drying step 3, and drying step 2 described above as one cycle. The textile product for evaluation after a total of 3 cycles were finished was used to evaluate water absorbency as shown below.

The mass of the textile product for evaluation was a value determined under an environment of 20°C and 43% RH in any of the steps.

(3b) Water absorbency evaluation

One fabric piece of 25 cm in length and 2.5 cm in width was cut out from the plain-woven portion of a cotton towel as the textile product for evaluation. One piece was cut out each from two cotton towels. A point 2 cm from the

end of a short side along the long side direction was marked with a black aqueous marker. The point marked was set as 0 cm, and a mark was placed every 1 cm along the long side direction up to 20 cm. The fabric piece was placed with the direction in which the mark of 0 cm was placed downward, the opposite short side downward, and the long side direction perpendicular to the water surface. Then, 2 L of tap water at 25°C was placed into a plastic beaker (capacity: 2 liters), and the short side of the fabric piece (lower end) was immersed in the water until the water surface reached the mark of 0 cm. The time at which the water surface reached 0 cm was taken as 0 minute, and the height of water in 15 minutes was measured. The top end to which the black mark blurred to cause the black ink to develop by chromatography was taken as the height of water. The average value of two fabric pieces (water absorption height 1) is shown in Table 4. Additionally, the height of water of Reference Example 3 (reference 3), which is a treatment method in which a piece is treated with no anionic surfactant and with no finishing agent composition for textile products, was designated "water absorption height 2". Water absorption height 1/water absorption height 2 is shown in Table 4.

[Table 4]

| | | | | | Reference Example 3 (reference 3) | Test group 1 | | Test group 2 | | Test group 3 | |
|---|---|---|---|---|---|---|---|---|---|---|---|
| | | | | | | Example 7 | Reference Example 4 (reference 4) | Example 8 | Reference Example 5 (reference 5) | Example 9 | Reference Example 6 (reference 6) |
| Step 1 | Treating liquid 1 | Composition (mg/kg) | (A) | (a-2) | | 170 | | 170 | | 170 | |
| | | | | Water | Balance (water only) | Balance | Balance (water only) | Balance | Balance (water only) | Balance | Balance (water only) |
| | | pH of treating liquid 1 (25°C) | | | | 7.0 | 7.0 | 7.0 | 7.0 | 7.0 | 7.0 |
| | | Temperature of treating liquid 1 (°C) | | | | 25 | 25 | 25 | 25 | 25 | 25 |
| Step 2 | Treating liquid 2 | Composition (mg/kg) | (B) | (b1-1) | | 40 | 40 | | | | |
| | | | | (b1-2) | | | | 40 | 40 | | |
| | | | | (b1-3) | | | | | | 40 | 40 |
| | | | | Water | Balance (water only) | Balance | Balance | Balance | Balance | Balance | Balance |
| | | pH of treating liquid 2 (25°C) | | | 7.0 | 7.0 | 7.0 | 7.0 | 7.0 | 7.0 | 7.0 |
| | | Temperature of treating liquid 2 (°C) | | | 25 | 25 | 25 | 25 | 25 | 25 | 25 |
| Evaluation | | Water absorbency (water absorption height in 15 minutes) | | Measurement value (cm) | 12.2 | 9.4 | 2.5 | 9.8 | 3.4 | 10.1 | 8.6 |
| | | | | Relative value* | - | 376 | 100 | 288 | 100 | 117 | 100 |
| | | Water absorption height 1 /water absorption height 2 (water absorption height 2: reference 3) | | | 1.0 | 0.77 | 0.20 | 0.80 | 0.28 | 0.83 | 0.70 |

*Relative value: value with the reference in each test group taken as 100

Finishing agent compositions for textile products containing a quaternary ammonium salt having different proportion of saturated hydrocarbon groups in the hydrocarbon group are known to each have different water absorbency. As shown in Table 4, however, treatment by using component (A) of the present invention allows the cotton towel to have excellent water absorbency even though the proportion of the saturated hydrocarbon group in the hydrocarbon group of the quaternary ammonium salt changes. Thus, the range of choices for types of finishing agent composition for textile products is extended. In a quaternary ammonium salt having a hydrocarbon group, an effect of improving the water absorbency of the present invention is anticipated as the proportion of the saturated hydrocarbon group in the hydrocarbon group increases. With respect to the effect of improving the water absorbency, for example, in comparison between Example 7 and Reference Example 3 (reference 3) in test group 1, the relative value of the water absorbency of Example 7 was 376 when the value of the water absorbency of Reference Example 3 (reference 3) was taken as 100. Similarly, it can be seen that it is possible to enhance the water absorbency in Examples in contrast to Reference Example in the same test group in the table. In the present invention, this relative value was used as a measure representing the effect of improving the water absorbency. A larger relative value indicates that the effect of improving the water absorbency is higher.

Water absorption height 1/water absorption height 2, as the ratio of water absorption height 1 of Example 7, Example 8, or Example 9 to water absorption height 2 of Reference Example 6 (reference 6), is 0.72 or more in each of the Examples, an excellent effect of retaining the water absorbency being exhibited.

[Examples 10 to 12, Comparative Examples 3 to 5, and Reference Example 7]

The softness, water absorbency, and detergency in the case of treatment of a textile product with treating liquid 1 and treating liquid 2 shown in Table 5 were evaluated. Evaluation was conducted in the same manner except that the concentration of component (A) in treating liquid 1, the pH and temperature of treating liquid 1, the concentration of component (B) of treating liquid 2, and the pH and temperature of treating liquid 2 were changed in <Evaluation of softness and water absorbency> of Examples 1 to 6. In (4a) water absorbency evaluation, the height of water of each of Examples and Comparative Examples was designated as "water absorption height 1". Additionally, the height of water of Reference Example 7 (reference 7), which is a treatment method in which a piece is treated with no anionic surfactant and with no finishing agent composition for textile products, was designated as "water absorption height 2". The value of water absorption height 1/water absorption height 2, as the ratio of water absorption height 1 of Examples 10 to 12 and Comparative Examples 3 to 5 to water absorption height 2, was calculated. Water absorption height 1/water absorption height 2 corresponds to water absorbency 1/water absorbency 2. A value of 0.72 or more is acceptable, and a value closer to 1 is preferable. The results are shown in Table 5.

[Table 5]

| | | | | | Example | | | Comparative Example | | | Reference Example |
|---|---|---|---|---|---|---|---|---|---|---|---|
| | | | | | 10 | 11 | 12 | 3 | 4 | 5 | 7 (reference 7) |
| Step 1 | Treating liquid 1 | Composition (mg/kg) | (A) | (a-2) | 250 | 250 | 250 | | | | |
| | | | | Water | Balance | Balance | Balance | Balance (water only) | Balance (water only) | Balance (water only) | Balance (water only) |
| | | pH of treating liquid 1 (25°C) | | | 7.3 | 7.3 | 7.3 | 7.3 | 7.3 | 7.3 | 7.3 |
| | | Temperature of treating liquid 1 (°C) | | | 15 | 15 | 15 | 15 | 15 | 15 | 15 |
| Step 2 | Treating liquid 2 | Composition (mg/kg) | (B) | (b1-2) | 15 | 15 | 15 | 15 | 15 | 15 | |
| | | | | (b3-1) | 100 | | | 100 | | | |
| | | | | (b4-1) | | 100 | | | 100 | | |
| | | | | (b6-1) | | | 100 | | | 100 | |
| | | | | Water | Balance | Balance | Balance | Balance | Balance | Balance | Balance (water only) |
| | | pH of treating liquid 2 (25°C) | | | 7.2 | 7.2 | 7.2 | 7.2 | 7.2 | 7.2 | 7.2 |
| | | Temperature of treating liquid 2 (°C) | | | 15 | 15 | 15 | 15 | 15 | 15 | 15 |
| Evaluation | | Water absorption height 1 (water absorption height in 15 minutes, cm) | | | 8.0 | 7.8 | 8.2 | 5.2 | 3.8 | 4.5 | 8.7 |
| | | Water absorption height 1/water absorption height 2 (water absorption height 2: reference 7) | | | 0.92 | 0.90 | 0.94 | 0.60 | 0.44 | 0.52 | 1.0 |

Water absorption height 1/water absorption height 2, as the ratio of the water absorption height of the knitted cotton cloth each of Examples 10 to 12, which was washed with component (A) of the present invention and then contacted with and treated with the finishing agent composition for textile products, that is, water absorption height 1 of Examples 10 to 12 to water absorption height 2 of the knitted cotton cloth treated in Reference Example 7 (reference 7), which is a treatment method in which the fabric is treated with no anionic surfactant and with no finishing agent composition for textile products, was 0.72 or more and close to 1 in each of the Examples, each providing substantially comparable water absorbency. The water absorbency of the knitted cotton cloth that was washed with Comparative Example 2 in Table 2 and then contacted with and treated with the finishing agent composition for textile products was comparable with that of Comparative Example 1 (reference 1).

As for the softness felt when the knitted cotton cloth was touched by hand, the knitted cotton cloth treated in each of Examples 10 to 12 and Comparative Examples 3 to 5 was softer than the cotton knitted cotton cloth treated in Reference Example 7.

As for the detergency, as the results of visual observation of the degree of staining by the model sebum attached to the knitted cotton cloth after treatment in Examples 10 to 12, the degree of staining was lower in each of these Examples than that of the knitted cotton cloth after treated in Reference Example 7, and the stains attached to the knitted cotton cloth had been washed off.

[Example 13 to 18 and Reference Example 1]

The softness and water absorbency in the case of treatment of a textile product with treating liquid 1 and treating liquid 2 shown in Table 6 were evaluated. Evaluation was conducted in the same manner except that the concentration of component (A) in treating liquid 1 was changed in <Evaluation of softness and water absorbency> of Examples 1 to 6. In (4a) water absorbency evaluation, the height of water of each of Examples was designated as "water absorption height 1". Additionally, the height of water of Reference Example 1 (reference 2), which is a treatment method in which a piece is treated with no anionic surfactant and with no finishing agent composition for textile products, was designated as "water absorption height 2". The value of water absorption height 1/water absorption height 2, as the ratio of water absorption height 1 of Examples 13 to 18 to water absorption height 2, was calculated. Water absorption height 1/water absorption height 2 corresponds to water absorbency 1/water absorbency 2. A value of 0.72 or more is acceptable, and a value closer to 1 is preferable. The results are shown in Table 5.

[Table 6]

| | | | | | Example | | | | | | Reference Example |
|---|---|---|---|---|---|---|---|---|---|---|---|
| | | | | | 13 | 14 | 15 | 16 | 17 | 18 | 1 (reference 2) |
| Step 1 | Treating liquid 1 | Composition (mg/kg) | (A) | (a-2) | 30 | 70 | 100 | 300 | 500 | 1000 | |
| | | | | Water | Balance | Balance | Balance | Balance | Balance | Balance | Balance (water only) |
| | | pH of treating liquid 1 (25°C) | | | 7.0 | 7.0 | 7.0 | 7.0 | 7.0 | 7.0 | 7.0 |
| | | Temperature of treating liquid 1 (°C) | | | 25 | 25 | 25 | 25 | 25 | 25 | 25 |
| Step 2 | Treating liquid 2 | Composition (mg/kg) | (B) | (b1-1) | 40 | 40 | 40 | 40 | 40 | 40 | |
| | | | | (b2-1) | 5 | 5 | 5 | 5 | 5 | 5 | |
| | | | | Water | Balance | Balance | Balance | Balance | Balance | Balance | Balance (water only) |
| | | pH of treating liquid 2 (25°C) | | | 7.0 | 7.0 | 7.0 | 7.0 | 7.0 | 7.0 | 7.0 |
| | | Temperature of treating liquid 2 (°C) | | | 25 | 25 | 25 | 25 | 25 | 25 | 25 |
| Evaluation | | Water absorption height 1 (water absorption height in 15 minutes, cm) | | | 6.9 | 7.5 | 8.1 | 7.9 | 8.0 | 8.0 | 8.6 |
| | | Water absorption height 1/water absorption height 2 (water absorption height 2: reference 2) | | | 0.80 | 0.87 | 0.94 | 0.92 | 0.93 | 0.93 | 1.0 |

Water absorption height 1/water absorption height 2, as the ratio of water absorption height 1 of the knitted cotton cloth each of Examples 13 to 18, in which a textile product was treated using component (A) of the present invention and then contacted with and treated with the finishing agent composition for textile products, to water absorption height 2 of the knitted cotton cloth treated in Reference Example 1 (reference 2), which is a treatment method in which the fabric is treated with no anionic surfactant and with no finishing agent composition for textile products, was 0.72 or more and close to 1 in each of the Examples.

As for the softness, the softness felt when the knitted cotton cloth was touched by hand was equivalent to or greater than that of the cotton knitted cotton cloth treated in Reference Example 1.

Component (A) of the present invention has modified the function of the finishing base for textile products contained in the finishing agent composition for textile products.

## Claims

1. A method for treating a textile product, comprising the following step 1 and step 2:

   step 1: a step of treating the textile product by contacting the textile product with a treating liquid 1 obtained by mixing water and (A) an internal olefin sulfonate having 17 or more and 24 or less carbons [hereinafter, referred to as a component (A)]; and
   step 2: a step of treating the textile product treated in step 1 by contacting the textile product with a treating liquid 2 obtained by mixing water and a finishing agent composition for textile products.

2. The method for treating a textile product according to claim 1, wherein the component (A) is an internal olefin sulfonate obtained by using, as raw material, internal olefins including an internal olefin having 17 or more and 24 or less carbons with a double bond located at position 5 or higher.

3. The method for treating a textile product according to claim 2, wherein the component (A) is an internal olefin sulfonate obtained by using, as raw material, internal olefins containing 10% by mass or more and 60% by mass or less of the internal olefin having 17 or more and 24 or less carbons with a double bond located at position 5 or higher.

4. The method for treating a textile product according to any of claims 1 to 3, wherein the finishing agent composition for textile products contains (B) a finishing base for textile products [hereinafter, referred to as a component (B)].

5. The method for treating a textile product according to claim 4, wherein the component (B) is one or more components selected from (B1) cationic surfactants having a hydrocarbon group having 16 or more and 24 or less carbons, (B2) silicone compounds, (B3) fatty alcohols, (B4) fatty acid esters, (B5) fats and oils, and (B6) fatty acids or salts thereof.

6. The method for treating a textile product according to claim 5, wherein the component (B) is one or more components selected from (B1) the cationic surfactants having a hydrocarbon group having 16 or more and 24 or less carbons and (B2) the silicone compounds.

7. The method for treating a textile product according to any of claims 1 to 6, wherein the textile product is a textile product containing cotton.

8. A method for producing a textile product, comprising the following step 1 and step 2:

   step 1: a step of treating the textile product by contacting the textile product with a treating liquid 1 obtained by mixing water and (A) an internal olefin sulfonate having 17 or more and 24 or less carbons; and
   step 2: a step of treating the textile product treated in step 1 by contacting the textile product with a treating liquid 2 obtained by mixing water and a finishing agent composition for textile products.

9. A method for retaining water absorbency of a textile product, wherein, in treating the textile product by steps including the following step 1 and step 2, (A) an internal olefin sulfonate having 17 or more and 24 or less carbons is employed as an anionic surfactant of step 1:

   step 1: a step of treating the textile product by contacting the textile product with a treating liquid 1 obtained by mixing water and the anionic surfactant; and
   step 2: a step of treating the textile product treated in step 1 by contacting the textile product with a treating

liquid 2 obtained by mixing water and a finishing agent composition for textile products.

**10.** A water absorbency retaining agent for textile products, comprising (A) an internal olefin sulfonate having 17 or more and 24 or less carbons.

**11.** A method for modifying a function of a finishing base for textile products, wherein, in treating a textile product by steps including the following step 1 and step 2, (A) an internal olefin sulfonate having 17 or more and 24 or less carbons is employed as an anionic surfactant of step 1:

step 1: a step of treating the textile product by contacting the textile product with a treating liquid 1 obtained by mixing water and the anionic surfactant; and
step 2: a step of treating the textile product treated in step 1 by contacting the textile product with a treating liquid 2 obtained by mixing water and a finishing agent composition for textile products containing a finishing base for textile products.

**12.** A function modifier for a finishing base for textile products, comprising (A) an internal olefin sulfonate having 17 or more and 24 or less carbons.

## INTERNATIONAL SEARCH REPORT

International application No.
PCT/JP2017/046375

A. CLASSIFICATION OF SUBJECT MATTER
Int.Cl. D06M13/256(2006.01)i, C11D1/14(2006.01)i, D06L1/16(2006.01)i, D06M13/463(2006.01)i, D06M15/643(2006.01)i, C07C309/20(2006.01)n, D06M101/04(2006.01)n

According to International Patent Classification (IPC) or to both national classification and IPC

B. FIELDS SEARCHED

Minimum documentation searched (classification system followed by classification symbols)
Int.Cl. C11D1/00-19/00, D06M13/00-15/715, D06L1/16, C07C309/20, D06M101/04

Documentation searched other than minimum documentation to the extent that such documents are included in the fields searched

| | |
|---|---|
| Published examined utility model applications of Japan | 1922-1996 |
| Published unexamined utility model applications of Japan | 1971-2017 |
| Registered utility model specifications of Japan | 1996-2017 |
| Published registered utility model applications of Japan | 1994-2017 |

Electronic data base consulted during the international search (name of data base and, where practicable, search terms used)
JSTPlus/JMEDPlus/JST7580 (JDreamIII), CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 63-161078 A (LION CORP.) 04 July 1988, claims, page 1, lower right column, lines 3-4, page 2, upper right column, lines 7-11, page 2, lower right column, line 15 to page 3, upper left column, line 4, page 4, lower right column, lines 1-4 (Family: none) | 1-2, 4-12<br>2 |
| Y | JP 59-27995 A (LION CORP.) 14 February 1984, page 2, lower left column, lines 14-16, table 1 & US 4507223 A, column 2, lines 33-35, table 1 | 2 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 March 2018 (12.03.2018) | 20 March 2018 (20.03.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No.
PCT/JP2017/046375

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2625150 B2 (LION CORP.) 02 July 1997, table 4 & JP 1-272564 A | 2 |
| A | JP 2014-76988 A (KAO CORP.) 01 May 2014, paragraphs [0038], [0053], [0055] & EP 2899258 A1, paragraphs [0044], [0062], [0064] & WO 2014/046175 A1 & CN 104603251 A | 1-12 |
| A | WO 2014/046176 A1 (KAO CORP.) 27 March 2014, claims, paragraphs [0038], [0053], [0055] & EP 2899257 A1, claims, paragraphs [0044], [0064], [0066] & JP 2014-77126 A | 1-12 |
| A | JP 61-60796 A (LION CORP.) 28 March 1986, claims, page 2, upper left column, lines 3-6, page 3, upper right column, line 5 to lower left column, line 1, page 5, upper right column, lines 19-20, table 1 (Family: none) | 1-12 |
| A | JP 2003-81935 A (LION CORP.) 19 March 2003, claims, paragraphs [0037], [0046] (Family: none) | 1-12 |
| A | JP 3-126793 A (UNILEVER NV) 29 May 1991, claims, page 21, line 17 to page 22, line 8, table 2 & GB 2236538 A, claims, page 11, lines 7-17, table 2 & CA 2026746 A & KR 10-1995-0005380 B | 1-12 |
| A | EP 482687 A1 (SHELL INTERNATIONALE RESEARCH MAATSCHAPPIJ B. V.) 29 April 1992, claims, examples & GB 9023366 A | 1-12 |
| A | US 5078916 A (SHELL OIL COMPANY) 07 January 1992, claims, examples & EP 377261 A2 | 1-12 |
| P, A | JP 2017-214678 A (KAO CORP.) 07 December 2017, claims (Family: none) | 1-12 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2011047065 A
- JP 2014076988 A
- EP 377261 A
- JP 3126793 A
- JP 6123071 A
- JP 4306296 A
- JP 6041578 A
- JP 8099036 A
- JP 2007131989 A
- JP 2011063784 A


### Non-patent literature cited in the description


- *J. Am. Oil Chem. Soc.,* 1992, vol. 69, 39